(19)

**Europäisches Patentamt**
**European Patent Office**
**Office européen des brevets**

(11)  **EP 4 763 865 A1**

(12)  **EUROPEAN PATENT APPLICATION**
published in accordance with Art. 153(4) EPC

(43) Date of publication:
**24.06.2026  Bulletin 2026/26**

(21) Application number: **24853764.9**

(22) Date of filing: **13.08.2024**

(51) International Patent Classification (IPC):
*C07K 16/40* (2006.01)  *C12N 15/13* (2006.01)
*C12N 15/70* (2006.01)  *A61K 49/00* (2006.01)
*G01N 33/573* (2006.01)

(52) Cooperative Patent Classification (CPC):
**A61K 49/00; C07K 16/00; C07K 16/40;**
**C12N 15/70; G01N 33/573**

(86) International application number:
**PCT/CN2024/111721**

(87) International publication number:
**WO 2025/036355 (20.02.2025 Gazette 2025/08)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority:  **14.08.2023  CN 202311018785**

(71) Applicants:
• **Minghui Pharmaceutical (Hangzhou) Limited**
  **Hangzhou, Zhejiang 310018 (CN)**
• **Minghui Pharmaceutical (Shanghai) Limited**
  **Shanghai 201210 (CN)**

(72) Inventors:
• **LIU, Bo**
  **Zhejiang 310018 (CN)**
• **ZHU, Liyuan**
  **Shanghai 201210 (CN)**
• **CAO, Guoqing**
  **Shanghai 201210 (CN)**
• **SHI, Junwei**
  **Shanghai 201210 (CN)**
• **CAO, Yuting**
  **Zhejiang 310018 (CN)**
• **WENG, Xialian**
  **Zhejiang 310018 (CN)**

(74) Representative: **SONN Patentanwälte GmbH & Co**
**KG**
**Riemergasse 14**
**1010 Wien (AT)**

Remarks:
The complete document including Reference Table(s) and the Sequence Listing(s) can be downloaded from the EPO website

(54)  **ANTI-C-MET NANOBODY AND USE THEREOF**

(57)  A nanobody specifically binding to c-MET or an antigen-binding fragment thereof, a composition containing the nanobody or the antigen-binding fragment thereof, and a nucleic acid molecule encoding the nanobody or the antigen-binding fragment thereof. Also provided is a use of the nanobody or the antigen-binding fragment thereof in the treatment and diagnosis of diseases.

EP 4 763 865 A1

**Description**

**Cross-Reference to Related Application**

The present application is based on and claims priority to the Chinese application with application number CN 202311018785.0 (filed on August 14, 2023). The disclosure of the Chinese application is incorporated herein by reference in its entirety.

**Technical Field**

The present invention relates to a nanobody or antigen-binding fragment thereof capable of specifically binding to c-MET, a composition containing the nanobody or antigen-binding fragment thereof, and a nucleic acid molecule encoding the antibody or antigen-binding fragment thereof. Furthermore, the present invention relates to a use of the nanobody or antigen-binding fragment thereof in the treatment and diagnosis of diseases.

**Background Art**

Cellular-mesenchymal epithelial transition factor (c-MET), also known as hepatocyte growth factor receptor (HGFR), is a transmembrane receptor with autonomous phosphorylation activity encoded by the MET gene, and belongs to the tyrosine kinase receptor superfamily [1,2]. Structurally, c-MET consists of an $\alpha$-chain and a $\beta$-chain linked by disulfide bonds, and is divided into an extracellular domain, a transmembrane helical domain, and an intracellular domain. The extracellular domain of c-MET contains three different functional regions: a SEMA domain covering the entire $\alpha$-chain and part of the N-terminus of the $\beta$-chain, which is a key region for ligand binding [3]; a cystine-rich domain with four disulfide bonds; and a region containing four immunoglobulin molecules [4]. The intracellular domain of c-MET also consists of three regulatory regions: a juxtamembrane domain containing phosphate sites Tyr1003 and Ser985, which primarily negatively regulates c-Met signal transduction; a catalytic domain containing phosphorylation sites Tyr1234 and Tyr1235, which primarily undergoes autophosphorylation to activate downstream signals and positively regulates the catalytic activity of tyrosine kinases; and a C-terminal multifunctional binding region containing Tyr1349 and Tyr1356, which primarily recruits various protein factors and adaptor molecules from the cytoplasm and plays a role in signal transmission [5,6].

Hepatocyte growth factor (HGF) is the only known high-affinity ligand for c-MET [7]. Under normal physiological conditions, when HGF binds to c-MET, c-MET is activated by the formation of dimers and phosphorylation at several sites in the juxtamembrane region, activating a series of downstream signaling pathways, such as PI3K-Akt, Ras-MAPK, STAT-INK, etc., thereby exerting a series of biological effects such as promoting cell proliferation, cell growth, and angiogenesis [8]. Studies have found that abnormal activation of c-MET pathways can promote the proliferation, invasion and migration of tumor cells, ultimately driving the occurrence and development of malignant tumors. It has been reported that c-MET signaling pathways are abnormally regulated in various types of solid tumors, such as lung cancer, gastric cancer, liver cancer, breast cancer, skin cancer, and colorectal cancer, and play important roles in the liver metastasis of colorectal cancer, the formation, growth and metastasis of oral squamous cell carcinoma, the invasion and metastasis of breast cancer, ovarian cancer and gastric cancer, as well as the occurrence and development of liver cancer, lung cancer, and pancreatic cancer. Abnormal activation of c-MET pathways mainly includes three types: MET14 exon skipping mutation, MET amplification, and MET protein overexpression [9]. Among them, MET14 exon skipping mutation is considered an independent oncogenic driver gene for lung cancer, with an incidence of 2-3% in NSCLC patients, and as high as 31.8% in patients with pulmonary sarcomatoid carcinoma [9-11]. The incidence of MET high amplification in lung adenocarcinoma is 1.0%. Although the incidence of MET amplification is not high, it is often accompanied by strong MET protein expression and is also one of the factors for poor prognosis [12]. The incidence of MET protein overexpression in lung adenocarcinoma can be as high as 65%, but it is not a primary oncogenic driver. Instead, it more commonly arises as a secondary event following the activation of other driver genes, thereby promoting tumor growth [13].

Monoclonal antibodies targeting human c-MET, such as Telisotuzumab, have been reported. Antibody-drug conjugates based on this monoclonal antibody have shown good potential in the treatment of non-small cell lung cancer patients. However, the need in this field for anti-tumor methods targeting c-MET with superior properties remains unmet.

**Contents of the Invention**

Through extensive research, the inventors of the present application have screened and obtained a humanized nanobody and antigen-binding fragment thereof capable of specifically recognizing the extracellular region of c-MET. Furthermore, the alpaca-derived nanobody used in the present invention has a molecular weight only half that of conventional antibodies (150 kDa), and can penetrate deeper into tumor core, thereby maximizing the opportunity for antibody drugs to bind to tumor cells and producing better therapeutic effects.

Nanobody and antigen-binding fragment thereof

In one aspect, the present invention provides a nanobody or antigen-binding fragment thereof capable of specifically binding to c-MET. The nanobody described herein typically consists of four framework regions (FRs) and three complementarity-determining regions (CDRs), referred to as FR1, CDR1, FR2, CDR2, FR3, CDR3, and FR4. The antigen-binding fragment comprises at least a portion of the nanobody, in which the portion is sufficient to endow the fragment with the ability to specifically bind to c-MET. In some embodiments, the nanobody of the present invention may be truncated at the N-terminus or C-terminus to contain only a portion of FR1 and/or FR4, or to lack one or two of those backbone regions, as long as it substantially maintains antigen-binding ability and specificity.

In some embodiments, the nanobody or antigen-binding fragment thereof comprises: CDR1, CDR2, and CDR3 contained in VHH as set forth in any one of SEQ ID NOs: 1, 14, and 15.

In some embodiments, the CDRs are defined according to the Kabat, Chothia, or IMGT numbering system.

In some embodiments, the nanobody or antigen-binding fragment thereof comprises: CDR1 as set forth in SEQ ID NO: 2, CDR2 as set forth in SEQ ID NO: 3, and CDR3 as set forth in SEQ ID NO: 4, wherein the CDRs are defined according to the Kabat numbering system.

In some embodiments, the nanobody or antigen-binding fragment thereof comprises: CDR1 as set forth in SEQ ID NO: 5, CDR2 as set forth in SEQ ID NO: 6, and CDR3 as set forth in SEQ ID NO: 7, wherein the CDRs are defined according to the Chothia numbering system.

In some embodiments, the nanobody or antigen-binding fragment thereof comprises: CDR1 as set forth in SEQ ID NO: 8, CDR2 as set forth in SEQ ID NO: 9, and CDR3 as set forth in SEQ ID NO: 10, wherein the CDRs are defined according to the IMGT numbering system.

In some embodiments, the nanobody or antigen-binding fragment thereof comprises a framework region sequence of a camel-derived antibody.

In some embodiments, the nanobody or antigen-binding fragment thereof comprises the sequence as set forth in SEQ ID NO: 1, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto. In some embodiments, the nanobody or antigen-binding fragment thereof comprises the sequence as set forth in SEQ ID NO: 1.

In some embodiments, the nanobody or antigen-binding fragment thereof is humanized, i.e., one or more of its framework regions have been substantially substituted by human framework regions.

In some embodiments, the nanobody or antigen-binding fragment thereof comprises a heavy chain framework region of a human immunoglobulin (e.g., a heavy chain framework region contained in the amino acid sequence encoded by a human heavy chain germline antibody gene), and the heavy chain framework region optionally comprises one or more (e.g., 1, 2, 3, 4, 5, 6, 7, 8, 9, or 10) reversion mutations from human residues to camel residues.

In some embodiments, the nanobody or antigen-binding fragment thereof comprises the sequence as set forth in SEQ ID NO: 14, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto. In some embodiments, the nanobody or antigen-binding fragment thereof comprises the sequence as set forth in SEQ ID NO: 14.

In some embodiments, the nanobody or antigen-binding fragment thereof comprises the sequence as set forth in SEQ ID NO: 15, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto. In some embodiments, the nanobody or antigen-binding fragment thereof comprises the sequence as set forth in SEQ ID NO: 15.

In some embodiments, the nanobody or antigen-binding fragment thereof is capable of specifically binding to human and/or cynomolgus c-MET.

Polypeptide construct

In another aspect, the present invention also provides a polypeptide construct capable of specifically binding to c-MET, which comprises the nanobody or antigen-binding fragment thereof of the present invention, and an immunoglobulin Fc domain.

Hereinafter, the Fc domain, also referred to as the Fc region, refers to a portion of the heavy chain constant region comprising CH2 and CH3. In some embodiments, the Fc domain comprises a hinge, CH2, and CH3. When the Fc domain comprises a hinge, the hinge regulates dimerization between two Fc-containing polypeptides. The Fc domain can be any antibody heavy chain constant region isotype. In some embodiments, the Fc domain is IgG1, IgG2, IgG3, or IgG4.

In some embodiments, the Fc domain contained in the polypeptide construct of the present invention is a native Fc region, which comprises an amino acid sequence consistent with the amino acid sequence of Fc region found in nature. For example, the Fc domain can be a native sequence human IgG1 Fc region, a native sequence human IgG2 Fc region, a native sequence human IgG3 Fc region, or a native sequence human IgG4 Fc region. The native Fc region may have effector functions. Exemplary "effector functions" include binding to Fc receptors; Clq binding and complement-dependent cytotoxicity (CDC); antibody-dependent cell-mediated cytotoxicity (ADCC); phage activity; downregulation of cell surface receptors (e.g., B cell receptors); and B cell activation, etc. Functional alterations can be achieved by replacing at least one amino acid residue in the native Fc region with a different residue or by chemical modification. For example, altering the antibody's affinity for effector ligands (e.g., FcR or complement C1q) can change (e.g., reduce or enhance) effector function.

Therefore, in some embodiments, the Fc domain contained in the polypeptide construct of the present invention may also be a variant Fc region, which may contain one or more (e.g., 1 to 10, e.g., 1 to 5) amino acid mutations or chemical modifications as compared to the native Fc region to alter one or more of the following properties of the antibody of the present invention: Fc receptor binding, antibody glycosylation, number of cysteine residues, effector cell function, or complement function, etc. In some embodiments, the Fc domain contained in the polypeptide construct of the present invention has a reduced or removed effector function, for example, an Fc domain containing a LALA mutation.

In some embodiments, the immunoglobulin Fc domain is optionally linked to the N-terminus and/or C-terminus (e.g., C-terminus) of the nanobody or antigen-binding fragment thereof via a peptide linker.

In some embodiments, the immunoglobulin Fc domain is an Fc domain of IgG (e.g., an Fc domain of IgG1, IgG2, IgG3, or IgG4).

In some embodiments, the immunoglobulin Fc domain is an Fc domain of human immunoglobulins, such as an Fc domain of human IgG (e.g., an Fc domain of human IgG1, IgG2, IgG3, or IgG4).

In some embodiments, the immunoglobulin Fc domain comprises the sequence as set forth in SEQ ID NO: 16, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto. In some embodiments, the immunoglobulin Fc domain comprises the sequence as set forth in SEQ ID NO: 16.

In some embodiments, the polypeptide construct comprises the sequence as set forth in any one of SEQ ID NOs: 11-13, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto.

Preparation of nanobody and polypeptide construct

The nanobody or polypeptide construct of the present invention can be prepared using various methods known in the art, such as by genetic engineering recombination techniques. For example, a DNA molecule encoding the nanobody or polypeptide construct of the present invention can be obtained by chemical synthesis or PCR amplification. The resulting DNA molecule is inserted into an expression vector and then transfected into a host cell. The transfected host cell is then cultured under specific conditions to express the antibody or polypeptide construct of the present invention.

In another aspect, the present invention also provides an isolated nucleic acid molecule, which encodes the nanobody or antigen-binding fragment thereof of the present invention, or the polypeptide construct of the present invention.

In another aspect, the present invention also provides a vector, which comprises the nucleic acid molecule of the present invention. In some embodiments, the vector is a cloning vector or an expression vector.

In another aspect, the present invention also provides a host cell, which comprises the nucleic acid molecule or vector of the present invention. Such host cell includes, but is not limited to, prokaryotic cell such as bacterial cell (e.g., *Escherichia coli* cell), and eukaryotic cell such as fungal cell (e.g., yeast cell), insect cell, plant cell, and animal cell (e.g., mammalian cell, such as mouse cell, human cell, etc.).

In another aspect, the present invention also provides a method for preparing the nanobody or antigen-binding fragment thereof of the present invention or the polypeptide construct of the present invention, which comprises culturing the host cell of the present invention under conditions allowing protein expression, and recovering the nanobody or antigen-binding fragment thereof or the polypeptide construct from the cultured host cell culture.

Bispecific or multispecific antibody

In another aspect, the present invention also provides a bispecific or multispecific antibody, which comprises the nanobody or antigen-binding fragment thereof of the present invention or the polypeptide construct of the present invention. The present invention also provides a use of the nanobody or antigen-binding fragment thereof or the polypeptide construct, or the nucleic acid molecule encoding the same, the vector, or the host cell of the present invention, in the manufacture of a bispecific or multispecific antibody.

In some embodiments, the bispecific or multispecific antibody is capable of specifically binding to c-MET, and additionally specifically binding to one or more other targets.

In some embodiments, the bispecific or multispecific antibody further comprises at least one secondary antibody having a second binding specificity against a second target.

Conjugate

In another aspect, the present invention also provides a conjugate, which comprises the nanobody or antigen-binding fragment thereof of the present invention, or the polypeptide construct of the present invention, or the bispecific or multispecific antibody of the present invention, and a conjugate moiety linked thereto.

In some embodiments, the conjugate moiety is selected from therapeutic agents (e.g., cytotoxic agents, cytokines, toxins, or radionuclide).

Pharmaceutical composition

In another aspect, the present invention also provides a pharmaceutical composition, which comprises the nanobody or antigen-binding fragment thereof, polypeptide construct, isolated nucleic acid molecule, vector, host cell, bispecific or multispecific antibody, or conjugate of the present invention, and a pharmaceutically acceptable carrier and/or excipient.

In some embodiments, the pharmaceutical composition further comprises an additional pharmaceutically active agent, such as an antitumor agent.

In some embodiments, in the pharmaceutical composition, the nanobody or antigen-binding fragment thereof, polypeptide construct, isolated nucleic acid molecule, vector, host cell, bispecific or multispecific antibody, or conjugate of the present invention, and the additional pharmaceutically active agent may be provided as separate components or as mixed components.

The nanobody or antigen-binding fragment thereof, polypeptide construct, isolated nucleic acid molecule, vector, host cell, bispecific or multispecific antibody, conjugate, or pharmaceutical composition of the present invention can be formulated into any dosage form known in the medical field, such as tablet, pill, suspension, emulsion, solution, gel, capsule, powder, granule, elixir, lozenge, suppository, injection (including injection solution, sterile powder for injection, and concentrated solution for injection), inhalant, spray, etc. Preferred dosage forms depend on the intended route of administration and therapeutic use. A preferred dosage form is an injection. Such injection can be a sterile injection solution. For example, the sterile injection solution can be prepared by incorporating an effective dose of the antibody or antigen-binding fragment of the present invention into a suitable solvent, and optionally, simultaneously incorporating other desired components (including, but not limited to, pH adjuster, surfactant, adjuvant, ionic strength enhancer, isotonic agent, preservative, diluent, or any combination thereof), followed by sterile filtration. Furthermore, the sterile injectable solution can be prepared as a sterile lyophilized powder (e.g., by vacuum drying or freeze-drying) for easy storage and use. Such sterile lyophilized powder can be dispersed in a suitable carrier prior to use.

In some exemplary embodiments, the pharmaceutically acceptable carrier and/or excipient comprises a sterile injectable liquid (e.g., an aqueous or non-aqueous suspension or solution). In some exemplary embodiments, such sterile injectable liquid is selected from water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution, and any combination thereof.

The pharmaceutical composition of the present invention may comprise a "therapeutically effective amount" or "prophylactically effective amount" of the nanobody or antigen-binding fragment thereof, isolated nucleic acid molecule, vector, host cell, bispecific or multispecific antibody, or conjugate as described in the present invention. The "prophylactically effective amount" refers to an amount sufficient to prevent, arrest, or delay the onset of a disease. The "therapeutically effective amount" refers to an amount sufficient to cure or at least partially arrest a disease and complications thereof in a patient already suffering from the disease. The therapeutically effective amount can vary depending on factors such as the severity of the disease to be treated, the overall state of the patient's own immune system, the patient's general characteristics such as age, weight, and gender, the method of administration, and other concurrent treatments, etc.

Pharmaceutical use

In another aspect, the present invention also provides a use of the nanobody or antigen-binding fragment thereof, polypeptide construct, isolated nucleic acid molecule, vector, host cell, bispecific or multispecific antibody, conjugate, or pharmaceutical composition of the present invention in the manufacture of a medicament for the prevention and/or treatment of a tumor in a subject.

In some embodiments, the tumor is a c-MET-positive tumor. Herein, c-MET positivity may be determined by immunohistochemistry and staining intensity evaluation.

In some embodiments, the tumor is a tumor with abnormal activation of c-MET pathway.

In some embodiments, the tumor is selected from the group consisting of solid tumors, such as gastric cancer, lung cancer (e.g., non-small cell lung cancer), liver cancer, head and neck cancer, skin cancer, colorectal cancer, ovarian cancer, esophageal cancer, pancreatic cancer, cervical cancer, mesothelioma, breast cancer, colorectal cancer, or oral squamous cell carcinoma.

In some embodiments, the subject is a mammal, such as a human.

In some embodiments, the nanobody or antigen-binding fragment thereof, polypeptide construct, isolated nucleic acid molecule, vector, host cell, bispecific or multispecific antibody, conjugate, or pharmaceutical composition is used alone or in combination with an additional pharmaceutically active agent (e.g., an antitumor agent).

Method for preventing and/or treating tumor

In another aspect, the present invention also provides a method for preventing and/or treating a tumor in a subject, comprising: administering to the subject in need thereof an effective amount of the nanobody or antigen-binding fragment thereof, polypeptide construct, isolated nucleic acid molecule, vector, host cell, bispecific or multispecific antibody, conjugate, or pharmaceutical composition of the present invention.

In some embodiments, the tumor is a c-MET-positive tumor.

In some embodiments, the tumor is a tumor with abnormal activation of c-MET pathway.

In some embodiments, the tumor is selected from the group consisting of solid tumors, such as gastric cancer, lung cancer (e.g., non-small cell lung cancer), liver cancer, head and neck cancer, skin cancer, colorectal cancer, ovarian cancer, esophageal cancer, pancreatic cancer, cervical cancer, mesothelioma, breast cancer, colorectal cancer, or oral squamous cell carcinoma.

In some embodiments, the subject is a mammal, such as a human.

The nanobody or antigen-binding fragment thereof, polypeptide construct, bispecific or multispecific antibody, conjugate, or pharmaceutical composition of the present invention can be formulated into any dosage form known in the medical field, such as tablet, pill, suspension, emulsion, solution, gel, capsule, powder, granule, elixir, lozenge, suppository, injection (including injection solution, sterile powder for injection, and concentrated solution for injection), inhaler, spray, etc. Preferred dosage forms depend on the intended route of administration and therapeutic use. The nanobody or antigen-binding fragment thereof, polypeptide construct, bispecific or multispecific antibody, conjugate, or pharmaceutical composition of the present invention shall be sterile and stable under the conditions of production and storage. A preferred dosage form is an injection. Such injection may be a sterile injectable solution. For example, the sterile injectable solution can be prepared by incorporating an effective dose of the nanobody or antigen-binding fragment, polypeptide construct, bispecific or multispecific antibody, conjugate, or pharmaceutical composition of the present invention into a suitable solvent, and optionally, simultaneously incorporating other desired components (including, but not limited to, pH adjuster, surfactant, adjuvant, ionic strength enhancer, isotonic agent, preservative, diluent, or any combination thereof), followed by sterile filtration. Furthermore, the sterile injectable solution can be prepared as a sterile lyophilized powder (e.g., by vacuum drying or freeze-drying) for easy storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution, and any combination thereof.

The nanobody or antigen-binding fragment, polypeptide construct, bispecific or multispecific antibody, conjugate, or pharmaceutical composition of the present invention can be administered by any suitable method known in the art, including but not limited to oral, buccal, sublingual, ocular, topical, parenteral, rectal, intrathecal, intracytoplasmic reticulum groove, groin, intravesical, topical (e.g., powder, ointment, or drops), or nasal routes. However, for many therapeutic uses, the preferred route of administration is parenteral (e.g., intravenous injection or bolus, subcutaneous injection, intraperitoneal injection, intramuscular injection). Those skilled in the art will understand that the route and/or method of administration will vary depending on the intended purpose. In some embodiments, the nanobody or antigen-binding fragment thereof, polypeptide construct, bispecific or multispecific antibody, conjugate, or pharmaceutical composition of the present invention is administered via intravenous injection or bolus injection.

Detection use

Kit

In another aspect, the present invention also provides a kit, which comprises the nanobody or antigen-binding fragment thereof of the present invention, or polypeptide construct of the present invention.
In some embodiments, the kit comprises a conjugate, wherein the conjugate comprises the nanobody or antigen-binding fragment thereof of the present invention, or polypeptide construct of the present invention, and a detectable label, such as an enzyme (e.g., horseradish peroxidase or alkaline phosphatase), a chemiluminescent reagent (e.g., acridinium ester, luminol and its derivative, or ruthenium derivative), a fluorescent dye (e.g., fluorescein or fluorescent protein), a radionuclide, or biotin, which is linked to the nanobody or antigen-binding fragment thereof or the polypeptide construct.
In some embodiments, the kit comprises the nanobody or antigen-binding fragment thereof of the present invention or the polypeptide construct of the present invention, and a secondary antibody capable of specifically recognizing the nanobody or antigen-binding fragment thereof or the polypeptide construct; optionally, the secondary antibody further comprises a detectable label, such as an enzyme (e.g., horseradish peroxidase or alkaline phosphatase), a chemiluminescent reagent (e.g., acridinium ester, luminol and its derivative, or ruthenium derivative), a fluorescent dye (e.g., fluorescein or fluorescent protein), a radionuclide, or biotin.

Detection method

In another aspect, the present invention also provides a method for detecting the presence or level of c-MET in a sample, which comprises using the nanobody or antigen-binding fragment thereof of the present invention or the polypeptide construct of the present invention. In some embodiments, the method is used for therapeutic purposes, diagnostic purposes, or non-therapeutic and non-diagnostic purposes.
In some embodiments, the method is an immunological assay, such as Western blotting, enzyme immunoassay (e.g., ELISA), chemiluminescent immunoassay, fluorescence immunoassay, or radioimmunoassay.
In some embodiments, the method comprises using a conjugate, wherein the conjugate comprises the nanobody or antigen-binding fragment thereof of the present invention, or the polypeptide construct of the present invention, and a detectable label, such as an enzyme (e.g., horseradish peroxidase or alkaline phosphatase), a chemiluminescent reagent (e.g., acridinium ester, luminol and its derivative, or ruthenium derivative), a fluorescent dye (e.g., fluorescein or fluorescent protein), a radionuclide, or biotin, which is linked to the nanobody or antigen-binding fragment thereof, or the polypeptide construct.
In some embodiments, the method comprises using the nanobody or antigen-binding fragment thereof of the present invention, or the polypeptide construct of the present invention, and the method further comprises using a secondary antibody carrying a detectable label (e.g., an enzyme (e.g., horseradish peroxidase or alkaline phosphatase), a chemiluminescent reagent (e.g., acridinium ester, luminol and its derivative, or ruthenium derivative), a fluorescent dye (e.g., fluorescein or fluorescent protein), a radionuclide, or biotin) to detect the nanobody or antigen-binding fragment thereof, or the polypeptide construct.
In some embodiments, the method comprises: (1) contacting the sample with the nanobody or antigen-binding fragment thereof of the present invention, the polypeptide construct of the present invention, or a conjugate comprising the nanobody or antigen-binding fragment thereof of the present invention, or the polypeptide construct of the present invention, and a detectable label, such as an enzyme (e.g., horseradish peroxidase or alkaline phosphatase), a chemiluminescent reagent (e.g., acridinium ester, luminol and its derivative, or ruthenium derivative), a fluorescent dye (e.g., fluorescein or fluorescent protein), a radionuclide, or biotin, which is linked to the nanobody or antigen-binding fragment thereof or the polypeptide construct; and, (2) detecting the formation of an antigen-antibody immune complex, or detecting the amount of the immune complex. The formation of the immune complex indicates the presence of c-MET or c-MET-expressing cells.
In some embodiments, the method is used to detect whether a tumor can be treated with an antitumor therapy targeting c-MET. In such embodiments, the sample is obtained from a subject who has a tumor, is suspected of having a tumor, or is at risk of having a tumor. In some embodiments, the sample is a cell sample (e.g., a sample containing tumor cells) or a body fluid sample (e.g., blood) from a subject (e.g., a mammal, such as a human). In some embodiments, the presence of c-MET or c-MET-expressing cells, and/or an increase in the amount of c-MET or c-MET-expressing cells as compared to a reference level (e.g., compared to a patient without tumor disease), indicates that the subject is suitable for c-MET-targeted antitumor therapy.

Use in manufacture of detection reagent

In another aspect, the present invention also provides a use of the nanobody or antigen-binding fragment thereof of the present invention, or the polypeptide construct of the invention, in the manufacture of a detection reagent for detecting the presence or level of c-MET in a sample or for detecting whether a tumor can be treated with c-MET-targeted antitumor therapy.

In some embodiments, the detection reagent detects the presence or level of c-MET in a sample and optionally detects whether a tumor can be treated with c-MET-targeted antitumor therapy by the method of the present invention as described above.

In some embodiments, the sample is a cell sample (e.g., a sample containing tumor cells) or a body fluid sample (e.g., blood) from a subject (e.g., a mammal, such as a human).

Terminology definition

In the present invention, unless otherwise stated, the scientific and technical terms used herein have the meanings commonly understood by those skilled in the art. Furthermore, the procedures used herein, such as those of cell culture, molecular biology, biochemistry, nucleic acid chemistry, and immunology, are all conventional procedures widely used in their respective fields. To better understand the present invention, definitions and explanations of relevant terms are provided below.

As used herein, the term "cellular-mesenchymal epithelial transition factor (c-MET)" refers to a transmembrane receptor with autonomous phosphorylation activity encoded by the MET gene, belonging to the tyrosine kinase receptor superfamily, whose sequence is well-known to those skilled in the art (see, for example, NCBI GENBANK database accession number: AAI30421.1). c-MET can bind to hepatocyte growth factor (HGF) and activate multiple important downstream signaling pathways such as PI3K-Akt, Ras-MAPK, and STAT-INK. Normal expression of the c-MET pathway promotes tissue differentiation and repair, while abnormal regulation promotes tumor cell proliferation and metastasis.

As used herein, the term "abnormal activation of c-MET pathway" has a meaning well-known to those skilled in the art, including MET14 exon skipping mutation, MET amplification, and/or MET protein overexpression. Abnormal activation of c-MET pathway occurs in various types of solid tumors, including but not limited to gastric cancer, lung cancer, liver cancer, head and neck cancer, skin cancer, colorectal cancer, ovarian cancer, esophageal cancer, pancreatic cancer, cervical cancer, mesothelioma, breast cancer, colorectal cancer, and oral squamous cell carcinoma.

As used herein, the term "camel-derived antibody" refers to an antibody against an antigen produced by *Camelidae* animals (including camel, alpaca, and L.glama) after immunization or antigen invasion. Those skilled in the art know that among the antibodies produced by *Camelidae* animals, there exists a light chain-lacking "heavy chain antibody" (Camelid heavy-chain antibody, HCAb). This kind of antibody contains only one heavy chain variable region (variable domain of heavy chain of HCAb, VHH) and two conventional regions, CH2 and CH3. The VHH region, when cloned and expressed separately, exhibits excellent structural stability and antigen-binding activity. The VHH is currently the smallest known unit capable of binding to a target antigen.

As used herein, the term "nanobody" has the meaning commonly understood by those skilled in the art, and refers to an antibody fragment consisting of a single monomeric variable antibody domain (e.g., a single heavy chain variable region), typically derived from the variable region of a heavy chain antibody (e.g., a camelid or shark antibody). Typically, a nanobody consists of four framework regions and three complementarity-determining regions, having a structure of FR1-CDR1-FR2-CDR2-FR3-CDR3-FR4. Nanobody can be truncated at the N-terminus or C-terminus to contain only a portion of FR1 and/or FR4, or to lack one or two of those framework regions, as long as it substantially maintains antigen-binding ability and specificity. Nanobody is also called single-domain antibody (sdAb), and the two terms are used interchangeably.

As used herein, the term "antigen-binding fragment" of a nanobody refers to a polypeptide containing a fragment of the nanobody, which retains the ability to specifically bind to the same antigen bound by the nanobody, and/or competes with the nanobody for specific binding to the antigen; it is also referred to as "antigen-binding moiety." See also Fundamental Immunology, Ch. 7 (Paul, W., ed., 2nd edition, Raven Press, N.Y. (1989)), which is incorporated herein by reference in its entirety for all purposes. The antigen-binding fragment of the antibody of the present invention can be generated by recombinant DNA technology or by enzymatic or chemical cleavage of the nanobody of the present invention. In some embodiments, the "antigen-binding fragment" of the nanobody may be truncated at the N-terminus or C-terminus as compared to the full-length nanobody to contain only a portion of FR1 and/or FR4, or lack one or two of those framework regions, as long as it substantially maintains antigen-binding ability and specificity.

The antigen-binding fragments of nanobody can be obtained from a given nanobody (e.g., the nanobody provided by the present invention) using conventional techniques known to those skilled in the art (e.g., recombinant DNA technology or enzymatic or chemical cleavage methods), and the antigen-binding fragments of nanobody can be screened for specificity in the same manner as for the intact nanobody.

As used herein, unless the context clearly indicates otherwise, when referring to the term "nanobody," it includes not only whole nanobody but also antigen-binding fragments of the nanobody.

As used herein, the term "complementarity-determining region" or "CDR" refers to the amino acid residues in the variable region of an antibody responsible for antigen binding. The nanobody contains three CDRs, named CDR1, CDR2, and CDR3. The precise boundaries of these CDRs can be defined according to various numbering systems known in the art, such as the IMGT numbering system (Lefranc et al., Dev. Comparat. Immunol. 27:55-77, 2003), the Kabat numbering system (Kabat et al., Sequences of Proteins of Immunological Interest, 5th Ed. Public Health Service, National Institutes of Health, Bethesda, Md., 1991), or the Chothia numbering system (Chothia & Lesk (1987) J. Mol. Biol. 196:901-917; Chothia et al. (1989) Nature 342:878-883). For a given nanobody, those skilled in the art will readily identify the CDRs defined by various numbering systems. Furthermore, the correspondences between different numbering systems are well known to those skilled in the art (for example, see Lefranc et al., Dev. Comparat. Immunol. 27:55-77, 2003).

In the present invention, the CDRs contained in the nanobody or antigen-binding fragment thereof of the present invention can be determined according to various numbering systems known in the art. In some embodiments, the CDRs contained in the nanobody or antigen-binding fragment thereof of the present invention are preferably determined by the IMGT, Kabat, or Chothia numbering systems.

As used herein, the term "framework region" or "FR" residues refers to those amino acid residues in the variable region of the antibody other than the CDR residues as defined above.

As used herein, the term "Fc domain" or "Fc region" refers to a portion of the heavy chain constant region containing CH2 and CH3. The Fc fragment of the antibody has a variety of different functions but does not participate in antigen binding. "Effector functions" mediated by the Fc region include Fc receptor binding; Clq binding and complement-dependent cytotoxicity (CDC); antibody-dependent cell-mediated cytotoxicity (ADCC); phage activity; downregulation of cell surface receptors (e.g., B cell receptors); and B cell activation. In some embodiments, the Fc region comprises a hinge, CH2, and CH3. When the Fc region comprises a hinge, the hinge regulates dimerization between two Fc-containing polypeptides. The Fc region can be any antibody heavy chain constant region isotype, such as IgG1, IgG2, IgG3, or IgG4.

The Fc domains can include both native Fc regions and variant Fc regions. Native Fc regions contain an amino acid sequence that is consistent with those found in naturally occurring Fc regions, for example, native sequence human Fc regions include native sequence human IgG1 Fc regions (non-A and A allotypes); native human sequence IgG2 Fc regions; native sequence human IgG3 Fc regions; and native sequence human IgG4 Fc regions, as well as their naturally occurring variants. Variant Fc regions contain an amino acid sequence that differs from the amino acid sequences of native sequence Fc regions due to at least one amino acid modification. In some embodiments, the variant Fc regions may possess an altered effector function (e.g., Fc receptor binding, antibody glycosylation, number of cysteine residues, effector cell function, or complement function) as compared to the native Fc regions.

As used herein, the term "humanized antibody" refers to a genetically engineered non-human antibody whose amino acid sequence is modified to increase sequence homology with human antibodies. Typically, all or part of the CDR regions of a humanized antibody are derived from a non-human antibody (donor antibody), and all or part of the non-CDR regions (e.g., variable FR regions and/or constant regions) are derived from a human immunoglobulin (receptor antibody). In some embodiments, the CDR regions of a humanized antibody are derived from a non-human antibody (donor antibody), and all or part of the non-CDR regions (e.g., variable FR regions and/or constant regions) are derived from a human immunoglobulin (receptor antibody). Humanized antibodies typically retain the intended properties of the donor antibody, including but not limited to antigen specificity, affinity, and reactivity. In the present application, the donor antibody may be a camel-derived antibody with the desired properties (e.g., antigen specificity, affinity, reactivity, etc.). To prepare a humanized antibody, the CDR regions of an immunized animal can be inserted into a human framework sequence using methods known in the art. In the context of nanobody, a humanized antibody may refer to a humanized VHH, i.e., a VHH in which one or more framework regions have been substantially replaced by human framework regions. In some cases, certain framework regions (FRs) of human immunoglobulin are replaced by corresponding non-human residues. Furthermore, a humanized VHH may contain residues not found in the initial VHH or human framework sequence but included to further improve and optimize the performance of the VHH or VHH-containing polypeptides.

As used herein, the term "specific binding" refers to a non-random binding reaction between two molecules, such as the reaction between an antibody and its targeted antigen. The strength or affinity of a specific binding interaction can be expressed by the equilibrium dissociation constant ($K_D$) of that interaction. In the present invention, the term "$K_D$" refers to the dissociation equilibrium constant of a specific antibody-antigen interaction, which describes the binding affinity between the antibody and the antigen. A smaller equilibrium dissociation constant indicates a tighter antibody-antigen binding and a higher affinity between the antibody and the antigen.

The specific binding properties between the two molecules can be determined using methods known in the art. One method involves measuring the rate of formation and dissociation of the antigen binding site/antigen complex. Both the "association rate constant" (ka or kon) and the "dissociation rate constant" (kdis or koff) can be calculated from the concentrations and the actual rates of association and dissociation (see Malmqvist M, Nature, 1993, 361:186-187). The ratio of kdis/kon is equal to the dissociation constant $K_D$ (see Davies et al., Annual Rev Biochem, 1990; 59:439-473). $K_D$, kon, and kdis values can be measured using any effective methods, such as surface plasmon resonance (SPR) in Biacore to measure the dissociation constant, or bioluminescent interferometry or Kinexa to measure the dissociation constant.

As used herein, the term "vector" refers to a nucleic acid delivery vehicle into which a polynucleotide can be inserted. When a vector enables the expression of a protein encoded by the inserted polynucleotide, it is called an expression vector. Vectors can be introduced into host cells through transformation, transduction, or transfection, enabling the expression of the genetic material elements they carry within the host cells. Vectors are well known to those skilled in the art and include, but are not limited to: plasmids; phage particles; Cos plasmids; artificial chromosomes, such as yeast artificial chromosome (YAC), bacterial artificial chromosome (BAC), or P1-derived artificial chromosome (PAC); bacteriophages such as λ phage or M13 phage, and animal viruses, etc. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, and papovavirus (e.g., SV40). A vector may contain multiple elements controlling expression, including but not limited to promoter sequence, transcription initiation sequence, enhancer sequence, selection element, and reporter gene. Additionally, the vector may contain a replication origin.

As used herein, the term "host cell" refers to a cell that can be used to introduce the vector, including but not limited to, prokaryotic cells such as *Escherichia coli* or *Bacillus subtilis,* fungal cells such as yeast cell or *Aspergillus,* insect cells such as S2 *Drosophila* cell or Sf9, or animal cells such as fibroblast, CHO cell, COS cell, NSO cell, HeLa cell, BHK cell, HEK 293 cell, or human cell.

As used herein, the term "identity" refers to the sequence matching between two polypeptides or two nucleic acids. When a position in two compared sequences is occupied by the same base or amino acid monomer subunit (e.g., a position in each of two DNA molecules is occupied by adenine, or a position in each of two polypeptides is occupied by lysine), then the molecules are identical at that position. The "percentage identity" between two sequences is a function of the number of matching positions shared by the two sequences divided by the number of positions being compared $\times$ 100. For example, if six out of ten positions in two sequences match, then the two sequences have an identity of 60%. For example, the DNA sequences CTGACT and CAGGTT have an identity of 50% (three out of six positions match). Typically, two sequences are aligned to produce the maximum identity when they are compared. Such alignment can be achieved by the method of Needleman et al. (1970) J. Mol. Biol. 48:443-453, readily using, for example, computer programs such as the Align program (DNAstar, Inc.). The percentage identity between two amino acid sequences can also be determined using the algorithm of E. Meyers and W. Miller (Comput. Appl. Biosci., 4:11-17 (1988)), which has been integrated into the ALIGN program (version 2.0), with a PAM120 weight residue table, a gap length penalty of 12, and a gap penalty of 4. In addition, the percentage identity between two amino acid sequences can be determined using the algorithm of Needleman and Wunsch (J Mol Biol. 48:444-453 (1970)), which is integrated into the GAP program within the GCG software package (available at www.gcg.com), with the Blossum 62 matrix or PAM250 matrix, and gap weights of 16, 14, 12, 10, 8, 6, or 4, along with length weights of 1, 2, 3, 4, 5, or 6.

As used herein, the term "conservative substitution" refers to an amino acid substitution that does not adversely affect or alter the intended properties of a protein/peptide containing the amino acid sequence. For example, conservative substitutions can be introduced using standard techniques known in the art, such as site-directed mutagenesis and PCR-mediated mutagenesis. Conservative amino acid substitutions include substitutions of amino acid residues with amino acid residues having similar side chains, such as substitutions with residues that are physically or functionally similar to the corresponding amino acid residues (e.g., having similar size, shape, charge, chemical properties, including the ability to form covalent or hydrogen bonds). Families of amino acid residues with similar side chains have been defined in the art. These families include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine), and aromatic side chains (e.g., tyrosine, phenylalanine, tryptophan, and histidine). Therefore, it is preferable to replace the corresponding amino acid residue with another amino acid residue from the same side chain family. Furthermore, amino acid residues can also be classified into categories defined by optional physical and functional properties. For example, there are alcohol group-containing residues (S and T), aliphatic residues (I, L, V and M), cycloalkenyl-related residues (F, H, W and Y), hydrophobic residues (A, C, F, G, H, I, L, M, R, T, V, W and Y), negatively charged residues (D and E), polar residues (C, D, E, H, K, N, Q, R, S and T), positively charged residues (H, K and R), small residues (A, C, D, G, N, P, S, T and V), very small residues (A, G and S), residues involved in corner formation (A, C, D, E, G, H, K, N, Q, R, S, P and T), and flexible residues (Q, T, K, S, G, P, D, E and R). Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al., Proc. Natl Acad. Set USA 94:412-417 (1997), which are incorporated herein by reference).

The twenty common amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. Furthermore, in the present invention, amino acids are generally represented by single-letter and three-letter abbreviations well known in the art. For example, alanine can be represented as A or Ala.

As used herein, the term "pharmaceutically acceptable carrier and/or excipient" refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995), and includes, but is not limited to: pH adjuster, surfactant, adjuvant, ionic strength enhancer, diluent, agent for maintaining osmotic pressure, agent for delaying absorption, and preservative. For example, the pH adjuster includes, but is not limited to, phosphate buffer. The surfactant includes, but is not limited to, cationic, anionic, or nonionic surfactant, such as Tween-80. The ionic strength enhancer includes, but is not limited to, sodium chloride. The preservative includes, but is not limited to, various antibacterial and antifungal agents, such as paraben, chlorobutanol, phenol, sorbic acid, etc. The agent for maintaining osmotic pressure includes, but is not limited to, sugar, NaCl, and analogue thereof. The agent for delaying absorption includes, but is not limited to, monostearate and gelatin. The diluent includes, but is not limited to, water, aqueous buffer (e.g., buffered saline), alcohols, and polyol (e.g., glycerol). The stabilizer has the meaning commonly understood by those skilled in the art, and is capable of stabilizing the desired activity of the active ingredient in the pharmaceutical product, including but not limited to monosodium glutamate, gelatin, SPGA, sugar (e.g., sorbitol, mannitol, starch, sucrose, lactose, dextran, or glucose), amino acid (e.g., glutamic acid, glycine), protein (e.g., dried whey, albumin, or casein) or degradation product thereof (e.g., lactalbumin hydrolysate). In some exemplary embodiments, the pharmaceutically acceptable carrier or excipient includes sterile injectable liquid (e.g., aqueous or non-aqueous suspension or solution). In some exemplary embodiments, such sterile injectable liquid is selected from water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution, and any combination thereof.

As used herein, the term "prevention" refers to a method implemented to prevent or delay the occurrence of a disease or condition or symptom (e.g., a tumor) in a subject. As used herein, the term "treatment" refers to a method implemented to obtain a beneficial or desired clinical outcome. For the purposes of the present invention, beneficial or desired clinical outcomes include, but are not limited to, alleviating symptoms, reducing the extent of disease, stabilizing (i.e., no longer worsening) the state of disease, delaying or slowing the progression of disease, improving or alleviating the state of disease, and relieving symptoms (whether partial or complete), whether detectable or undetectable. Furthermore, "treatment" can also refer to prolonged survival as compared to expected survival (if no treatment was received).

As used herein, the term "subject" refers to a mammal, such as a human, cynomolgus, or mouse. In some embodiments, the subject (e.g., a human, cynomolgus, or mouse) has a disease associated with c-MET (e.g., c-MET-positive tumor) or is at risk of having such a disease.

As used herein, the term "effective amount" refers to an amount sufficient to obtain or at least partially obtain the desired effect. For example, an effective amount for preventing disease (e.g., involving c-MET-positive tumor) refers to an amount sufficient to prevent, arrest, or delay the onset of the disease; an effective amount for treating disease refers to an amount sufficient to cure or at least partially stop the disease and complications thereof in a patient already suffering from the disease. Determining such an effective amount is entirely within the capabilities of those skilled in the art. For example, an effective amount for therapeutic use will depend on the severity of the disease to be treated, the overall state of the patient's own immune system, the patient's general characteristics such as age, weight, and gender, the manner of administration, and other concurrent treatments, etc.

As used herein, the term "antibody-mediated endocytosis" refers to the phenomenon of an antibody crossing the cell membrane after binding to a cell surface antigen.

As used herein, the terms "cancer" and "tumor" are used interchangeably and refer to a broad class of diseases characterized by the uncontrolled growth of abnormal cells in the body. Uncontrolled cell division can lead to the formation of malignant tumors or cells that invade adjacent tissues and may metastasize to distant sites of the body via the lymphatic system or bloodstream. The cancer includes benign and malignant cancers, as well as dormant tumors or micrometastases. The cancer also includes hematologic malignancies.

Beneficial effect of the invention

The nanobody of the present invention can specifically recognize c-MET and possess good endocytic activity and endocytosis-mediated cell-killing activity, showing good potential for the treatment of tumors (especially tumors expressing c-MET). Furthermore, the humanized nanobody of the present invention not only retains the function and properties of the parent antibody but also has a high degree of humanization, thus allowing safe administration to human subjects without inducing immunogenic reactions. In addition, the nanobody of the present invention has a molecular weight only half that of conventional antibodies, allowing it to penetrate deeper into the tumor core, maximizing the opportunity for antibody drugs to bind to tumor cells and producing better therapeutic effects. In summary, the nanobody of the present invention has significant clinical value.

The embodiments of the present invention will be described in detail below with reference to the accompanying drawings and examples. However, those skilled in the art will understand that the following drawings and examples are for illustrative purposes only and are not intended to limit the scope of the present invention. Various objects and advantages of the present invention will become apparent to those skilled in the art from the following detailed description of the drawings and preferred embodiments.

**Brief Description of the Drawings**

Fig. 1 shows the ELISA results of the binding of candidate antibodies to human c-MET antigen.

Fig. 2 shows the FACS results of the binding of candidate antibodies to human c-MET antigen.

Figs. 3A to 3B show the detection results of endocytic activity of candidate antibodies in Hs746T human gastric cancer cells endogenously expressing human c-MET. Fig. 3A shows the percentage curves of endocytosis at different time points; Fig. 3B shows the endocytosis results at 37°C for 4 hours.

Fig. 4 shows the Fab-ZAP method-based antibody endocytosis-mediated cell killing assay for antibody candidates.

Fig. 5 shows the FACS results of MHAB07-3 and humanized antibodies thereof binding to human c-MET antigen.

Fig. 6 shows the FACS results of MHAB07-3 and humanized antibodies thereof as well as positive control antibody binding to human c-MET antigen.

Figs. 7A to 7B shows the detection results of endocytic activity of MHAB07-3 and humanized antibodies thereof in Hs746T human gastric cancer cells endogenously expressing human c-MET. Fig. 7A shows the percentage curves of endocytosis at different time points; Fig. 7B shows the endocytosis results at 37°C for 4 hours.

Fig. 8 shows the Fab-ZAP method-based antibody endocytosis-mediated cell killing assay for MHAB07-3 and humanized antibodies thereof.

Fig. 9 shows the detection results of binding ability of MHAB07-3-2 to c-MET antigens of different species (human, cynomolgus, mouse, and rat).

Sequence Information

Table 1: The information of the sequences involved in the present invention is described in the table below:

| SEQ ID NO | Description | Sequence |
|---|---|---|
| 1 | MHAB07-3 VHH | QLQLVESGGGLVQAGGSLRLSCAASGRITNTYVMGWFRQAPGKERELQAAITWTVGSTYYVDSVKGRFTISRDNAKNTLYLQMNSLKPEDTAVYYCAASRFSAWPPRGTLGEWNSWGQGTQVTVSS |
| 2 | MHAB07-3 VHH-CDR1 (Kabat definition) | TYVMG |
| 3 | MHAB07-3 VHH-CDR2 (Kabat definition) | AITWTVGSTYYVDSVKG |
| 4 | MHAB07-3 VHH-CDR3 (Kabat definition) | SRFSAWPPRGTLGEWNS |
| 5 | MHAB07-3 VHH-CDR1 (Chothia definition) | GRITNTY |

(continued)

| SEQ ID NO | Description | Sequence |
|---|---|---|
| 6 | MHAB07-3 VHH-CDR2 (Chothia definition) | TWTVGS |
| 7 | MHAB07-3 VHH-CDR3 (Chothia definition) | SRFSAWPPRGTLGEWNS |
| 8 | MHAB07-3 VHH-CDR1 (IMGT definition) | GRITNTYV |
| 9 | MHAB07-3 VHH-CDR2 (IMGT definition) | ITWTVGST |
| 10 | MHAB07-3 VHH-CDR3 (IMGT definition) | AASRFSAWPPRGTLGEWNS |
| 11 | MHAB07-3 | QLQLVESGGGLVQAGGSLRLSCAASGRITNTYVMGWFR QAPGKERELQAAITWTVGSTYYVDSVKGRFTISRDNAK NTLYLQMNSLKPEDTAVYYCAASRFSAWPPRGTLGEW NSWGQGTQVTVSSEPKSSDKTHTCPPCPAPEAAGGPSVF LFPPKPKDTLMISRTPEVTCVVVDVSHEDPEVKFNWYV DGVEVHNAKTKPREEQYNSTYRVVSVLTVLHQDWLNG KEYKCKVSNKALPAPIEKTISKAKGQPREPQVYTLPPSRE EMTKNQVSLTCLVKGFYPSDIAVEWESNGQPENNYKTT PPVLDSDGSFFLYSKLTVDKSRWQQGNVFSCSVMHEAL HNHYTQKSLSLSPGK |
| 12 | MHAB07-3-1 | QLQLVESGGGLVQPGGSLRLSCAASGRITNTYVMGWVR QAPGKEREFQAAITWTVGSTYYVDSVKGRFTISRDNAK NTLYLQMNSLRPEDTAVYYCAASRFSAWPPRGTLGEW NSWGQGTQVTVSSEPKSSDKTHTCPPCPAPEAAGGPSVF LFPPKPKDTLMISRTPEVTCVVVDVSHEDPEVKFNWYV DGVEVHNAKTKPREEQYNSTYRVVSVLTVLHQDWLNG KEYKCKVSNKALPAPIEKTISKAKGQPREPQVYTLPPSRE EMTKNQVSLTCLVKGFYPSDIAVEWESNGQPENNYKTT PPVLDSDGSFFLYSKLTVDKSRWQQGNVFSCSVMHEAL HNHYTQKSLSLSPGK |
| 13 | MHAB07-3-2 | QLQLVESGGGLVQPGGSLRLSCAASGRITNTYVMGWVR QAPGKEREFVAAITWTVGSTYYVDSVKGRFTISRDNAK NTLYLQMNSLRPEDTAVYYCAASRFSAWPPRGTLGEW NSWGQGTQVTVSSEPKSSDKTHTCPPCPAPEAAGGPSVF LFPPKPKDTLMISRTPEVTCVVVDVSHEDPEVKFNWYV DGVEVHNAKTKPREEQYNSTYRVVSVLTVLHQDWLNG KEYKCKVSNKALPAPIEKTISKAKGQPREPQVYTLPPSRE EMTKNQVSLTCLVKGFYPSDIAVEWESNGQPENNYKTT PPVLDSDGSFFLYSKLTVDKSRWQQGNVFSCSVMHEAL HNHYTQKSLSLSPGK |

(continued)

| SEQ ID NO | Description | Sequence |
|---|---|---|
| 14 | MHAB07-3-1 VHH | QLQLVESGGGLVQPGGSLRLSCAASGRITNTYVMGWVR QAPGKEREFQAAITWTVGSTYYVDSVKGRFTISRDNAK NTLYLQMNSLRPEDTAVYYCAASRFSAWPPRGTLGEW NSWGQGTQVTVSS |
| 15 | MHAB07-3-2 VHH | QLQLVESGGGLVQPGGSLRLSCAASGRITNTYVMGWVR QAPGKEREFVAAITWTVGSTYYVDSVKGRFTISRDNAK NTLYLQMNSLRPEDTAVYYCAASRFSAWPPRGTLGEW NSWGQGTQVTVSS |
| 16 | Fc (LALA) of human IgG1 | EPKSSDKTHTCPPCPAPEAAGGPSVFLFPPKPKDTLMISR TPEVTCVVVDVSHEDPEVKFNWYVDGVEVHNAKTKPR EEQYNSTYRVVSVLTVLHQDWLNGKEYKCKVSNKALP APIEKTISKAKGQPREPQVYTLPPSREEMTKNQVSLTCLV KGFYPSDIAVEWESNGQPENNYKTTPPVLDSDGSFFLYS KLTVDKSRWQQGNVFSCSVMHEALHNHYTQKSLSLSPG K |

## Specific Models for Carrying Out the present invention

The present invention will now be described in the following non-limiting examples.

Those skilled in the art will understand that the examples are used to describe the present invention by the way of examples and are not intended to limit the scope sought to be protected by the present application. Unless otherwise specified, the experimental methods in the examples were conventional methods. Where specific conditions were not specified in the examples, conventional conditions or conditions recommended by the manufacturer were followed. Reagents or instruments whose manufacturers were not specified were all commercially available conventional products.

Example 1: Obtaining anti-human c-MET nanobody sequences

1-1. Screening and identification of specific nanobodies

Peripheral blood was collected from immunized alpacas, RNA was extracted, and cDNA samples were prepared. The VHH antibody encoding gene was cloned by PCR, and a single-domain antibody phage display library was constructed. Clones were randomly selected, and the library size and diversity were identified.

Using the c-MET antigen (ACRO, Cat. MET-H5227), the single-domain antibody phage display library was enriched through panning. Single clones were then selected for Phage-ELISA detection. Candidate clones with different amino acid sequences in CDR regions were selected, ultimately identifying 43 unique sequences.

1-2. Verification of unique sequence solubility and antibody expression

The plasmids of the 43 positive clones were extracted and separately transformed into E. *coli Rosetta,* induced overnight at 20°C and 200 rpm with 0.4 mM IPTG. On the next day, the cells were centrifuged, the precipitates were resuspended in PBS, disrupted by ultrasonication, and centrifuged again to obtain the supernatants from the 43 clones. The binding activity (OD450 values as shown in Table 2 below) of the supernatants from 43 disrupted clones to human c-MET antigen was detected by ELISA. Some clones showed negative results (this could be a false negative due to the inability of the anti-VHH secondary antibody to recognize the antigen, and required further verification, therefore these unique sequences were not directly eliminated in this screening step).

Table 2: Binding activity of 43 clones to human c-MET antigen

| Unique sequence | OD450 | Unique sequence | OD450 |
|---|---|---|---|
| MH172-HGF R-1-2 | 0.199 | MH172-HGF R-2-10 | 3.234 |
| MH172-HGF R-1-6 | 0.102 | MH172-HGF R-2-21 | 0.23 |
| MH172-HGF R-1-8 | 0.749 | MH172-HGF R-2-27 | 2.21 |
| MH172-HGF R-1-13 | 0.221 | MH172-HGF R-2-32 | 0.454 |
| MH172-HGF R-1-21 | 0.211 | MH172-HGF R-2-173 | 3.227 |
| MH172-HGF R-1-22 | 0.136 | MH172-HGF R-7-1-8 | 0.206 |
| MH172-HGF R-1-24 | 0.229 | MH172-HGF R-7-1-10 | 0.298 |
| MH172-HGF R-1-28 | 0.202 | MH172-HGF R-7-1-12 | 0.166 |
| MH172-HGF R-1-29 | 0.231 | MH172-HGF R-7-1-27 | 1.818 |
| MH172-HGF R-1-31 | 0.175 | MH172-HGF R-7-1-66 | 1.426 |
| MH172-HGF R-1-32 | 0.183 | MH172-HGF R-7-1-79 | 0.898 |
| MH172-HGF R-1-38 | 2.935 | MH172-HGF R-7-2-20 | 1.592 |
| MH172-HGF R-1-40 | 2.653 | MH172-HGF R-7-2-21 | 3.227 |
| MH172-HGF R-1-42 | 0.164 | MH172-HGF R-7-2-47 | 0.213 |
| MH172-HGF R-1-47 | 2.878 | MH172-HGF R-7-2-52 | 1.101 |
| MH172-HGF R-1-51 | 0.362 | MH172-HGF R-7-2-65 | 0.991 |
| MH172-HGF R-1-72 | 0.432 | MH172-HGF R-7-2-79 | 3.41 |
| MH172-HGF R-1-73 | 0.372 | MH172-HGF R-7-2-83 | 1.114 |
| MH172-HGF R-1-79 | 2.209 | MH172-HGF R-7-2-89 | 2.477 |
| MH172-HGF R-2-1 | 3.382 | MH172-HGF R-7-2-90 | 1.974 |
| MH172-HGF R-2-4 | 3.169 | MH172-HGF R-7-2-92 | 3.259 |
| MH172-HGF R-2-6 | 0.12 | PBS | 0.077 |

The unique sequences with strong binding activity to human c-MET, no cysteine residues (C) in the CDR3 region, and a theoretical PI value >7 were selected, and their VHH sequences were integrated into the Fc (LALA) sequence of human IgG1 (SEQ ID NO:16). CHO transient expression services were provided by Shanghai Baiying Biotechnology Co., Ltd., producing 18 MHB07 candidate antibodies (as shown in Table 3). After expression, the antibodies were purified with Protein A, and dissolved in PBS, and the antibody solutions had a purity (SEC, 280nm) >95%, and endotoxin (EU/mg) <1. After aliquoting, the antibodies were stored at -80°C.

Table 3: MHB07 candidate antibodies

| No. | Unique seq | OD450 | Unique seq/PBS |
|---|---|---|---|
| MHAB07 - 1 | MH172-7-H-2-79 | 3.41 | 44.28571429 |
| MHAB07 - 2 | MH172-H-2-1 | 3.382 | 43.92207792 |
| MHAB07 - 3 | MH172-7-H-2-92 | 3.259 | 42.32467532 |
| MHAB07 - 4 | MH172-7-H-2-21 | 3.227 | 41.90909091 |
| MHAB07 - 5 | MH172-H-2-4 | 3.169 | 41.15584416 |
| MHAB07 - 6 | MH172-H-2-27 | 2.21 | 28.7012987 |
| MHAB07 - 7 | MH172-7-H-2-90 | 1.974 | 25.63636364 |
| MHAB07 - 8 | MH172-7-H-2-20 | 1.592 | 20.67532468 |
| MHAB07 - 9 | MH172-7-H-1-66 | 1.426 | 18.51948052 |

(continued)

| No. | Unique seq | OD450 | Unique seq/PBS |
|---|---|---|---|
| MHAB07 - 10 | MH172-7-H-2-83 | 1.114 | 14.46753247 |
| MHAB07 - 11 | MH172-7-H-2-52 | 1.101 | 14.2987013 |
| MHAB07 - 12 | MH172-H-1-73 | 0.372 | 4.831168831 |
| MHAB07 - 13 | MH172-H-1-51 | 0.362 | 4.701298701 |
| MHAB07 - 14 | MH172-H-2-21 | 0.23 | 2.987012987 |
| MHAB07 - 15 | MH172-H-1-21 | 0.211 | 2.74025974 |
| MHAB07 - 16 | MH172-7-H-1-8 | 0.206 | 2.675324675 |
| MHAB07 - 17 | MH172-H-1-31 | 0.175 | 2.272727273 |
| MHAB07 - 18 | MH172-H-2-6 | 0.12 | 1.558441558 |
| | PBS | 0.077 | 1 |

Example 2: Purification and activity detection of antibodies

2-1. ELISA binding assay

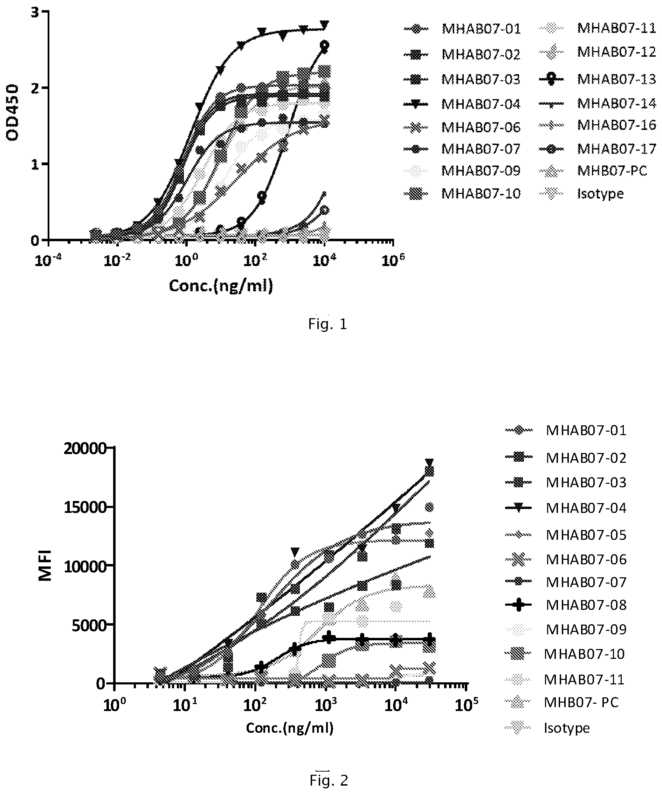

The binding ability of the MHB07 candidate antibodies (anti-human c-MET nanobodies) to human c-MET antigen was detected using ELISA. Human c-MET, His Tag (Sino Biological, Cat. No. 10692-H08H) was diluted to 0.5 µg/mL with PBS, added to an ELISA plate (Corning, Cat. No. 9018) at 100 µL/well, coated and incubated overnight at 4°C. The plate was washed three times with PBST, added with 200 µL of 3% BSA/PBST to each well, blocked at room temperature for 1 hour, then washed five times with PBST. Test antibodies (the numbers of test antibodies were MHAB07-01 to 14/16/17, the positive control antibody was MHB07-PC (Telisotuzumab), the initial concentration was 10 µg/mL, and 4-fold gradient dilution was performed) were added, incubated at room temperature for 1 hour, and then washed seven times with PBST to remove unbound antibodies. 100 µL of 1:50,000 diluted goat anti-human IgG Fc(HRP) (Abcam, Cat.No. ab97225) was added to each well, and incubated at room temperature for 30 minutes. The plate was washed seven times with PBST to remove excess secondary antibodies, and then 100 µL of 1-Step™ Ultra TMB-ELISA Substrate Solution (Absin, Cat. No. 9178) was added to each well and incubated at room temperature in the dark for 15 min for color development. Then, 100 µL of TMB stop solution (Absin, Cat. No. abs9472) was added to each well to terminate the reaction. The absorbance values at 450 nm were measured using a microplate reader, and four-parameter curves were plotted using GraphPad.
All experimental results are expressed as mean ± SEM (mean standard error). Prism 6 (GraphPad) software was used for graphing and data analysis.
The results are shown in Fig. 1 and Table 4. Among the MHB07 candidate antibodies, the 10 antibodies with the strongest binding activity to human c-MET antigen were MHAB07-01 to 11, and these 11 sequences were further analyzed by the following FACS binding activity assay.

Table 4: ELISA binding assay of MHB07 candidate antibodies

| | MHAB 07-1 | MHAB 07-2 | MHAB 07-3 | MHAB 07-4 | MHAB0 7-6 | MHAB 07-7 | MHAB 07-9 | MHAB 07-10 | MHAB 07-11 | MHAB 07-12 | MHAB 07-13 | MHAB 07-14 | MHAB 07-16 | MHAB 07-17 | MHB07 -PC | Isotype |
|---|---|---|---|---|---|---|---|---|---|---|---|---|---|---|---|---|
| Bottom | 0.05577 | 0.06358 | 0.05539 | 0.01914 | 0.002245 | 0.03806 | 0.05079 | 0.0394 | 0.04187 | 0.05681 | 0.05572 | 0.05673 | 0.05579 | 0.06309 | 0.04074 | 0.05819 |
| Top | 2.034 | 1.921 | 1.895 | 2.771 | 1.593 | 1.546 | 1.577 | 2.205 | 1.803 | 0.06641 | 2.81 | 1673 | 0.5013 | 0.748 | 2.024 | 0.07945 |
| EC50 (ng/ml) | 0.7951 | 0.8379 | 0.7886 | 1.321 | 28.38 | 1.045 | 13.09 | 10.17 | 3.028 | +infinit y | 884.5 | 239554 590 | ~ 20432 | 10538 | 9.25 | ~ 4086 |
| Isoelectr ic point | 7.68 | 8.29 | 8.28 | 7.69 | 7.18 | 6.43 | 6.73 | 8.28 | 8.04 | 6.29 | 7.31 | 8.44 | 7.68 | 8.26 | | |
| Expressi on level (mg/L) | 373 | 269.8 | 258.5 | 263 | 228 | 252.8 | 236.5 | 177 | 228 | 278.5 | 24 | 178 | 323 | 209 | | |
| Purity (SEC, 280nm) | 99.95% | 98.78% | 99.72% | 99.28% | 100.00% | 99.01% | 99.92% | 99.96 % | 99.02% | 99.44% | / | 99.96% | 99.08% | 98.22% | | |

EP 4 763 865 A1

## 2-2. FACS binding assay

In the FACS assay, Hs746T human gastric cancer cells endogenously expressing human c-MET (cultured in Hs746T cell complete culture medium: Dulbecco's Modified Eagle's Medium (ATCC, Cat. No. 30-2002) + 10% FBS (Sigma-Aldrich, Cat. No. F8687) + 1% P/S (Gibco, Cat. No. 15140-122)) were used to detect the binding of the candidate antibodies. The initial concentration of the candidate antibodies was 30 μg/mL, and then 3-fold gradient dilution was performed (30000 ng/mL, 10000 ng/mL, 3333.33 ng/mL, 1111.11 ng/mL, 370.37 ng/mL, 123.46 ng/mL, 41.15 ng/mL, and 13.72 ng/mL). FITC anti-human IgG Fc antibody secondary antibody (1:200, Biolegend, Cat. No. 410720) was added at 100 μL/well to detect the binding of the candidate antibodies. FITC fluorescence intensity (Mean Fluorescence Intensity, MFI) values were detected by flow cytometry.

All experimental results are expressed as mean ± SEM (mean standard error). Prism 6 (GraphPad) software was used for plotting and data analysis.

The FACS binding results are shown in Fig. 2 and Table 5. MHAB07-01/02/03/04/05 showed stronger binding activity to Hs746T than MHB07-PC. Further endocytosis assay and endocytosis-mediated cell-killing activity assay were performed on these five antibodies.

Table 5: FACS binding assay of MHB07 candidate antibodies

| | MHAB0 7-01 | MHAB07 -02 | MHAB0 7-03 | MHAB07-04 | MHAB0 7-05 | MHAB0 7-06 | MHAB0 7-07 | MHAB0 7-08 | MHAB0 7-09 | MHAB0 7-10 | MHAB0 7-11 | MHB07-PC | Isotype |
|---|---|---|---|---|---|---|---|---|---|---|---|---|---|
| Bottom | -419.5 | ~ -446767 | -6972 | ~ -48519 | 9.084 | 434.9 | 114.6 | 504.5 | -299.3 | -345.6 | 50.7 | 610 | -123.6 |
| Top | 13808 | ~ 29010 | 42900 | ~ 215489 | 12176 | 1309 | 511.2 | 3816 | 1201 | 3429 | 5249 | 8344 | 722.3 |
| EC50 (ng/ml) | 161 | ~ 0.0 | 41247 | ~ 4.381e+014 | 109.6 | ~ 9662 | ~ 46.48 | 208.7 | 11573 | 856.9 | ~ 414.0 | 787.9 | ~ 9745 |

2-3. Endocytosis assay

Hs746T human gastric cancer cells endogenously expressing human c-MET (cultured in complete Hs746T cell culture medium: Dulbecco's Modified Eagle's Medium (ATCC, Cat.No. 30-2002) + 10% FBS (Sigma-Aldrich, Cat.No. F8687) + 1% P/S (Gibco, Cat.No. 15140-122) were used to detect the endocytic activity of the candidate antibodies. $1 \times 10^6$/mL cell suspension and 10 $\mu$g/mL anti-c-MET candidate antibody solutions were prepared using FACS buffer (PBS containing 2% FBS). The cell suspension was added to a 15 mL EP tube, and centrifuged at 1200 rpm and 4°C for 3 min, and the supernatant was discarded. The cells were added with 2 mL of the candidate antibody solutions, respectively, resuspended and incubated on ice for 30 min, and then washed three times with pre-chilled FACS buffer. The cells were then resuspended in 2 mL of DMEM complete medium, mixed, and added to 96-well plates at 100 $\mu$L/well. The plates were separately incubated at 4°C and 37°C for 0 h, 1 h, 2 h, and 4 h (duplicates). After incubation at each time point, the cells were centrifuged at 1200 rpm and 4°C for 3 min. 100 $\mu$L of FITC anti-human IgG Fc antibody dilution buffer (1:200, Biolegend, Cat.no. 410720) was added to each well, and the cells were incubated at 4°C in the dark for 30 min. After washing away unbound secondary antibody, the FITC fluorescence intensity (Mean Fluorescence Intensity, MFI) was detected by flow cytometry, and the endocytosis rate was calculated using the following formula:

$$\text{Internalization } (\%) = 100\% - MFI_T / MFI_{T0} * 100\%.$$

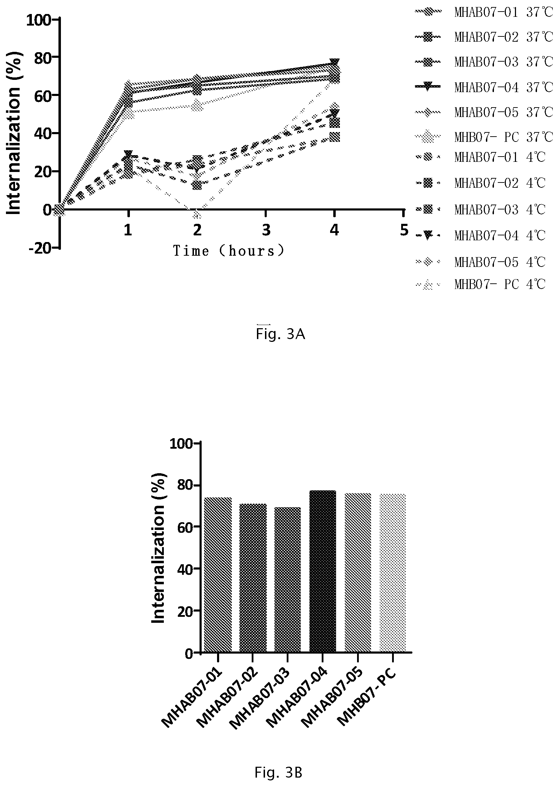

All experimental results are expressed as mean $\pm$ SEM (mean standard error). Prism 6 (GraphPad) software was used for plotting and data analysis.
The endocytosis percentage curves at each time point are shown in Fig. 3A. Fig. 3B shows the endocytosis results at 37°C for 4 h, indicating that MHAB07-01/02/03/04/05 exhibited good endocytic activity in Hs746T cells.

2-4. Detection of antibody endocytosis-mediated cell killing by Fab-ZAP method-based assay

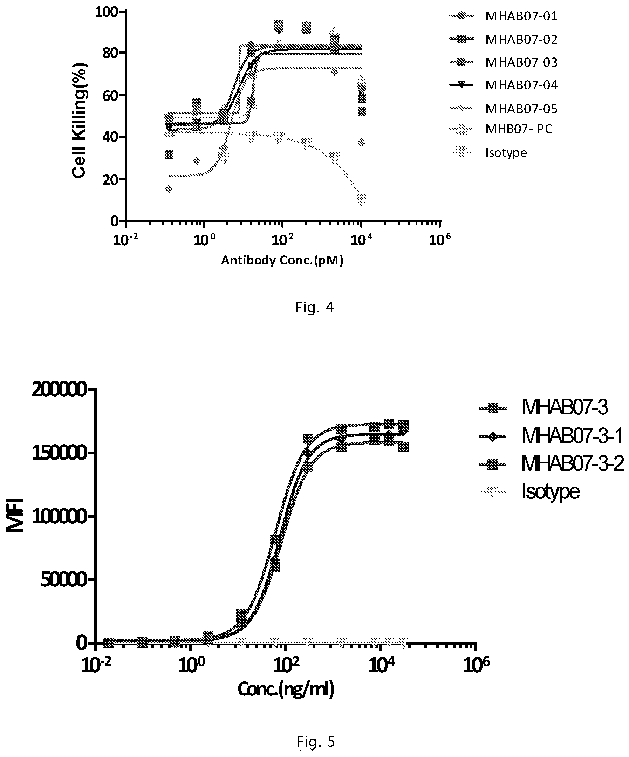

Hs746T human gastric cancer cells (cultured in Hs746T cell complete medium: Dulbecco's Modified Eagle's Medium (ATCC, Cat. No. 30-2002) + 10% FBS (Sigma-Aldrich, Cat. No. F8687) + 1% P/S (Gibco, Cat. No. 15140-122)) were digested, resuspended in complete medium, and counted. The cell density was adjusted to 4E4 cells/mL. The cells were added to each well of a 96-well cell plate at 50 $\mu$L (approximately 1500 cells) per well, and incubated at 37°C in a 5% $CO_2$ cell culture incubator for 16 hours. Diluted ZAP solution containing 9 nM ZAP-Fab was prepared using DMEM complete medium. The antibody was then serially diluted with the diluted ZAP solution to obtain 10 nM to 128 fM working solutions (8 concentration gradients, 1:5 dilution). After incubation at 37°C for 15 min, the working solutions were added to the cell culture plate at 50 $\mu$L per well, and mixed well, and then the plate was incubated in a 37°C cell culture incubator for 120 hours. The cell well that had not been treated with antibody-Fab-ZAP was used as the positive control, and 7.5 $\mu$L of Triton-X 100 was added to this well in advance followed by incubation for 30 min. The cell wells (NT) that had not been treated with antibody-Fab-ZAP or Triton-X 100 served as negative controls. Then, 20 $\mu$L of MTS (Promega, Cat: G3598B) was added to each well of the cell culture plate and incubated in a 37°C cell culture incubator for 2 hours. Data was read after shaking for 10 s in a microplate reader at wavelength A490. Cell killing efficiency was calculated using the formula: killing rate % = 100% - (OD$_{sample}$ - OD$_{Triton-X100}$) / (OD$_{NT}$ - OD$_{Triton-X100}$) * 100%.
All experimental results are expressed as mean $\pm$ SEM (mean standard error). Prism 6 (GraphPad) software was used for plotting and data analysis.
The endocytosis-mediated cell killing curves are shown in Fig. 4. The results in Fig. 4 and Table 6 indicate that the MHB07 candidate antibodies exhibited similar killing activity against Hs746T. Considering FACS binding, endocytosis results, and the degree of sequence humanization, the preferred antibody MHAB07-03 was obtained. Its VHH sequence is set forth in SEQ ID NO: 1, its CDR1 to CDR3 sequences according to Kabat definition are set forth in SEQ ID NOs: 2-4, its CDR1 to CDR3 sequences according to Chothia definition are set forth in SEQ ID NOs: 5-7, and its CDR1 to CDR3 sequences according to IMGT definition are set forth in SEQ ID NOs: 8-10. MHAB07-03 would be humanized and further screened.

Table 6: Detection of antibody endocytosis-mediated cell killing by Fab-ZAP method-based assay

| | MHAB07-01 | MHAB07-02 | MHAB07-03 | MHAB07-04 | MHAB07-05 | MHB07- PC | Isotype |
|---|---|---|---|---|---|---|---|
| Bottom | 51.44 | 43.65 | 46.82 | 45.46 | 21.43 | 49.69 | N/A |
| Top | 83.52 | 82.98 | 79.54 | 81.78 | 72.59 | 83.23 | N/A |
| EC50 (pM) | ~ 7.972 | 5.681 | ~ 17.44 | 7.915 | 4.879 | ~ 19.94 | N/A |

Example 3: Humanization and activity assay of anti-human c-MET nanobody

The VHH sequence of MHAB07-03 was humanized to obtain two humanized nanobodies: MHAB07-3-1 (with VHH as set forth in SEQ ID NO: 14) and MHAB07-3-2 (with VHH as set forth in SEQ ID NO: 15). According to the method in Example 1, the above VHH sequences were integrated into the Fc (LALA) sequence of human IgG1 (SEQ ID NO: 16), followed by purification and expression for subsequent activity assay.

3-1. FACS binding assay

3-1-1. FACS binding assay of anti-human c-MET nanobody and its humanized antibodies

In the FACS assay, the binding activity of MHAB07-3 and its humanized antibodies was detected using Hs746T gastric cancer cells endogenously expressing human c-MET (cultured in complete Hs746T cell culture medium: Dulbecco's Modified Eagle's Medium (ATCC, Cat. No. 30-2002) + 10% FBS (Sigma-Aldrich, Cat. No. F8687) + 1% P/S (Gibco, Cat. No. 15140-122)). The initial concentration of test antibodies was 30 μg/mL, and 2-fold gradient dilution and 5-fold gradient dilution were performed sequentially (30000 ng/mL, 15000 ng/mL, 7500 ng/mL, 1500 ng/mL, 300 ng/mL, 60 ng/mL, 12 ng/mL, 2.4 ng/mL, 0.48 ng/mL, 0.096 ng/mL, and 0.0192 ng/mL). The binding of the test antibodies was detected by adding Alexa Fluor® 488 AffiniPure goat anti-human IgG (H+L) secondary antibody (1:300, Yeasen, Cat:33126ES60) at 100 μL/well. FITC fluorescence intensity (Mean Fluorescence Intensity, MFI) was measured by flow cytometry.

All experimental results are expressed as mean ± SEM (mean standard error). Prism 6 (GraphPad) software was used for plotting and data analysis.

The binding curves are shown in Fig. 5. As can be seen from Fig. 5 and Table 7, the binding activity of MHAB07-3-1 and MHAB07-3-2 to human c-MET antigen was not significantly weakened as compared to parental MHAB07-3. Therefore, MHAB07-3-1 and MHAB07-3-2 were selected for subsequent endocytosis assay and endocytosis-mediated cell killing activity assay.

Table 7: FACS binding assay of anti-human c-MET nanobody and its humanized antibodies

|  | MHAB07-3 | MHAB07-3-1 | MHAB07-3-2 | Isotype |
|---|---|---|---|---|
| Bottom | 2173 | 2329 | 1758 | N/A |
| Top | 172721 | 164809 | 158478 | N/A |
| EC50 (ng/ml) | 62.32 | 77.68 | 82.92 | N/A |
| Isoelectric Point | 8.28 | 8.32 | 8.32 |  |
| Expression Level (mg/L) | 258.5 | 293 | 240 |  |
| Purity (SEC, 280nm) | 99.72% | 99.44% | 98.83% |  |

3-1-2. FACS binding assay of anti-human c-MET nanobody and its humanized antibodies relative to positive control antibody

In the FACS assay, the binding activity of MHAB07-3 and its humanized antibody MHAB07-3-2 relative to the positive control antibody MHB07-PC (Telisotuzumab) was detected using Hs746T gastric cancer cells endogenously expressing human c-MET (cultured in complete Hs746T cell culture medium: Dulbecco's Modified Eagle's Medium (ATCC, Cat. No. 30-2002) + 10% FBS (Sigma-Aldrich, Cat. No. F8687) + 1% P/S (Gibco, Cat. No. 15140-122)). The initial concentration of test antibodies was 30 μg/mL, and 4-fold gradient dilution was performed (30000 ng/mL, 7500 ng/mL, 1875 ng/mL, 468.75 ng/mL, 117.19 ng/mL, 29.30 ng/mL, 7.32 ng/mL, 1.83 ng/mL, 0.46 ng/mL, 0.11 ng/mL, 0.03 ng/mL, and 0.007 ng/mL), in total of 12 dilution points. Fluorescence secondary antibody (PE Goat anti-Human IgG Fc Secondary Antibody, Invitrogen, Cat. No.: 12-4998-82, 1:200 dilution) was added for staining at 100 μL/well to detect antibody binding. PE fluorescence intensity (Mean Fluorescence Intensity, MFI) was then detected by flow cytometry.

All experimental results are expressed as mean ± SEM (mean standard error). Prism 6 (GraphPad) software was used for plotting and data analysis.

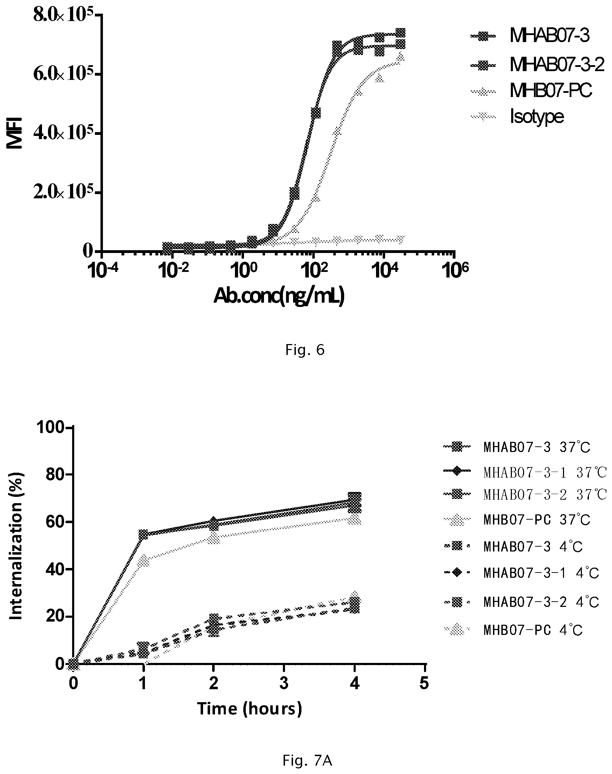

The binding curves are shown in Fig. 6. As shown in Fig. 6 and Table 8, MHAB07-3 and MHAB07-3-2 exhibited stronger binding activity to the human c-MET antigen on the surface of tumor cells than the positive control antibody MHB07-PC (Telisotuzumab).

Table 8: FACS binding assay of anti-human c-MET nanobody and its humanized antibodies relative to positive control antibody

|  | MHAB07-3 | MHAB07-3-2 | MHB07-PC | Isotype |
|---|---|---|---|---|
| Bottom | 13898 | 22682 | 18015 | N/A |
| Top | 735974 | 697025 | 647284 | N/A |
| EC50 (ng/ml) | 72.23 | 64.20 | 309.7 | N/A |

3-2. Endocytosis assay

The endocytic activity of the candidate antibodies was detected using Hs746T gastric cancer cells endogenously expressing human c-MET. $1 \times 10^6$/mL cell suspension and 10 $\mu$g/mL anti-c-MET candidate antibody solutions were prepared using FACS buffer (PBS containing 2% FBS). The cell suspension was added to a 15 mL EP tube, centrifuged at 1200 rpm and 4°C for 3 min, and the supernatant was discarded. The cells were added with 2 mL of the antibody solutions, respectively, resuspended, incubated on ice for 30 min, and then washed three times with pre-chilled FACS buffer. The cells were resuspended in 2 mL of DMEM complete medium, mixed well, and added to 96-well plates at 100 $\mu$L/well. The plates were incubated at 4°C and 37°C for 0 h, 1 h, 2 h, and 4 h (duplicates), respectively. After incubation at each time point, the cells were centrifuged at 1200 rpm and 4°C for 3 min. A diluted solution of Alexa Fluor® 488 AffiniPure goat anti-human IgG (H+L) secondary antibody (YEASEN, Cat.no. 33126ES60) was added at 100 $\mu$L per well, and incubated at 4°C for 30 min. After washing away unbound secondary antibody, the FITC fluorescence intensity (Mean Fluorescence Intensity, MFI) was detected by flow cytometry, and the endocytosis rate was calculated using the following formula:

$$\text{Internalization } (\%) = 100\% - \text{MFI}_T/\text{MFI}_{T0} * 100\%.$$

All experimental results are expressed as mean $\pm$ SEM (mean standard error). Prism 6 (GraphPad) software was used for plotting and data analysis.

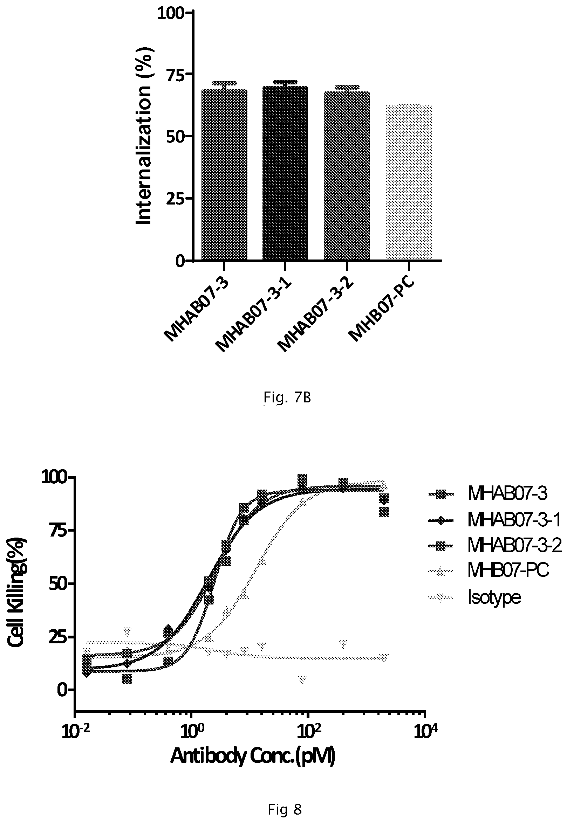

The endocytosis percentage curves are shown in Fig. 7A. As shown in Fig. 7B, after 4 hours of endocytosis at 37°C, MHAB07-3, MHAB07-3-1 and MHAB07-3-2 exhibited good endocytic activity in Hs746T cells. The endocytic activity of MHAB07-3-1 and MHAB07-3-2 was not significantly weakened as compared to parental MHAB07-3, and was stronger than that of MHB07-PC.

3-3. Detection of antibody endocytosis-mediated cell killing by Fab-ZAP method-based assay

Hs746T human gastric cancer cells cells (cultured in Hs746T cell complete medium: Dulbecco's Modified Eagle's Medium (ATCC, Cat. No. 30-2002) + 10% FBS (Sigma-Aldrich, Cat. No. F8687) + 1% P/S (Gibco, Cat. No. 15140-122)) were digested, resuspended in complete medium, and counted. The cell density was adjusted to 4E4 cells/mL. The cells were added to a 96-well cell plate at 50 $\mu$L (approximately 1500 cells) per well, and incubated at 37°C in a 5% $CO_2$ cell culture incubator for 16 hours. A diluted ZAP solution containing 9 nM ZAP-Fab was prepared using DMEM complete medium. The antibodies were then serially diluted with the diluted ZAP solution to obtain 4 nM to 32 fM (10 concentration gradients, 1:5 dilution for the first 5 points, and 1:2 dilution for the last 5 points) working solutions. After incubation at 37°C for 15 min, the working solutions were added to the cell culture plate at 50 $\mu$L per well, and mixed well, and then the cell culture plate was incubated at 37°C for 120 hours. The well that had not been treated with antibody-Fab-ZAP was used as a positive control, and added with 7.5 $\mu$L of Triton-X 100 in advance and incubated for 30 min. The wells that had not treated with antibody-Fab-ZAP or Triton-X 100 (NT) served as negative controls. Then, 20 $\mu$L of MTS (Promega, Cat: G3598B) was added to each well of the cell culture plate, and incubated in a 37°C cell culture incubator for 2 hours. Data were read after shaking for 10 seconds using a microplate reader, and the detection wavelength was A490. The cell killing efficiency was calculated using the formula: killing rate % = 100% - ($OD_{sample}$ - $OD_{Triton-X100}$) / ($OD_{NT}$ - $OD_{Triton-X100}$) * 100%.

All experimental results are expressed as mean $\pm$ SEM (mean standard error). Prism 6 (GraphPad) software was used for plotting and data analysis.

The endocytosis-mediated cell killing curves are shown in Fig. 8, and the results are summarized in Table 9. The results showed that MHAB07-3-1 and MHAB07-3-2 exhibited similar Fab-ZAP killing activity against Hs746T cells as MHAB07-3. Considering FACS binding, endocytosis results, and sequence humanization levels, MHAB07-3-1 demonstrated the highest level of humanization and exhibited good binding activity, endocytosis activity, and endocytosis-mediated cell killing.

Table 9: Detection of antibody endocytosis-mediated cell killing by Fab-ZAP method-based assay

|  | MHAB07-3 | MHAB07-3-1 | MHAB07-3-2 | MHB07-PC | Isotype |
|---|---|---|---|---|---|
| Bottom | 8.795 | 9.618 | 16.19 | 15.36 | N/A |
| Top | 93.84 | 94.42 | 95.93 | 98.35 | N/A |
| EC50 (pM) | 2.501 | 1.954 | 2.626 | 12.75 | N/A |

3-4. Cross-species activity

The binding ability of MHAB07-3-2 (anti-human c-MET nanobody) to c-MET antigens from different species (human, cynomolgus, mouse, and rat) was detected using ELISA. Human c-MET (Human c-MET, His Tag (Sino Biological, Cat. No. 10692-H08H)), cynomolgus c-MET (Cynomolgus, Rhesus c-MET Protein, His Tag (Sino Biological, Cat. No. 90304-C08H)), mouse c-MET (Mouse c-MET Protein, His Tag (Sino Biological, Cat. No. 50622-M08H)), and rat c-MET (Rat c-MET Protein, hFc Tag (Sino Biological, Cat. No. 80004-R02H)) were diluted to 0.5 µg/mL with PBS, respectively, then added to an ELISA plate (Corning, Cat. No. 9018) at 100 µL/well, coated and incubated overnight at 4°C. The plate was washed three times with PBST, added with 3% BSA/PBST at 200 µL per well, blocked at room temperature for 1 hour, then washed five times with PBST. The test antibodies were added (their initial concentration was 10 µg/mL, and 10-fold gradient dilution was performed), incubated at room temperature for 1 hour, then washed seven times with PBST to remove unbound antibodies. Goat anti-human IgG Fc(HRP) (Abcam, Cat.No. ab97225) diluted at 1:50,000 was added at 100 µL per well, rabbit anti-camelid VHH cocktail [HRP] (GenScript, A02016) diluted at 1:10,000 was added at 100 µL per well to the wells coated with Rat c-MET Protein, and incubation was carried out at room temperature for 30 min. The plate was washed seven times with PBST to remove excess secondary antibodies, then 100 µL of 1-Step™ Ultra TMB-ELISA Substrate Solution (Absin, Cat. No. 9178) was added to each well, and subjected to color development at room temperature in the dark for 15 min for color development. Then, 100 µL of TMB stop solution (Absin, Cat. No. abs9472) was added to each well to terminate the reaction. The absorbance values at 450 nm were read using a microplate reader, and four-parameter curves were plotted using GraphPad.

All experimental results are expressed as mean ± SEM (mean standard error). Prism 6 (GraphPad) software was used for plotting and data analysis.

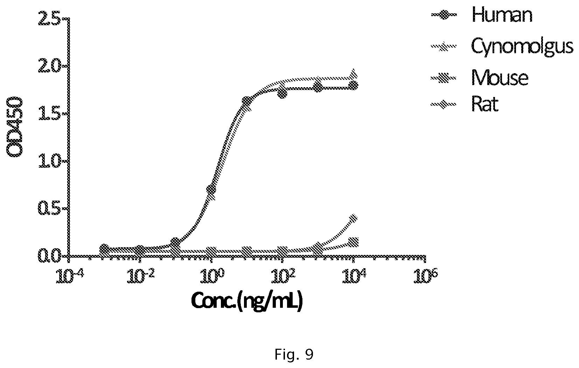

The results are shown in Fig. 9 and Table 10. MHAB07-3-2 bound to only human c-MET and cynomolgus c-MET, and showed comparable binding activity to human c-MET and cynomolgus c-MET. It did not bind to mouse c-MET or rat c-MET.

Table 10: Cross-species activity assay on c-MET antigens of human, cynomolgus, mouse, and rat

|  | MHAB07-3 | MHAB07-3-1 | MHAB07-3-2 | MHB07-PC | Isotype |
|---|---|---|---|---|---|
| Bottom | 8.795 | 9.618 | 16.19 | 15.36 | N/A |
| Top | 93.84 | 94.42 | 95.93 | 98.35 | N/A |
| EC50 (pM) | 2.501 | 1.954 | 2.626 | 12.75 | N/A |

Although specific embodiments of the present invention have been described in detail, those skilled in the art will understand that various modifications and variations can be made to the details based on all the disclosed teachings, and all such changes are within the scope of protection of the present invention. The full scope of the present invention is given by the appended claims and any equivalents thereof.

References

[1] Gherardi E, Birchmeier W, Birchmeier C, Vande Woude G. Targeting MET in cancer: rationale and progress. Nat Rev Cancer. 2012;12(2):89-103.

[2] Stoker M, Gherardi E, Perryman M, Gray J. Scatter factor is a fibroblast-derived modulator of epithelial cell mobility. Nature. 1987;327(6119):239-42.

[3] Kong-Beltran M, Stamos J, Wickramasinghe D. The Sema domain of Met is necessary for receptor dimerization and activation. Cancer Cell. 2004;6(1):75-84.

[4] Gherardi E, Sandin S, Petoukhov MV, Finch J, Youles ME, Ofverstedt LG, et al. Structural basis of hepatocyte growth factor/scatter factor and MET signaling. Proc Natl Acad Sci U S A. 2006;103(11):4046-51.

[5] Uchikawa E, Chen Z, Xiao GY, Zhang X, Bai XC. Structural basis of the activation of c-MET receptor. Nat Commun. 2021;12(1):4074.

[6] Ponzetto C, Bardelli A, Zhen Z, Maina F, dalla Zonca P, Giordano S, et al. A multifunctional docking site mediates signaling and transformation by the hepatocyte growth factor/scatter factor receptor family. Cell. 1994;77(2):261-71.

[7] Fasolo A, Sessa C, Gianni L, Broggini M. Seminars in clinical pharmacology: an introduction to MET inhibitors for the medical oncologist. Ann Oncol. 2013;24(1):14-20.

[8] Trusolino L, Bertotti A, Comoglio PM. MET signaling: principles and functions in development, organ regeneration and cancer. Nat Rev Mol Cell Biol. 2010;11(12):834-48.

[9] Drilon A, Clark JW, Weiss J, Ou SI, Camidge DR, Solomon BJ, et al. Antitumor activity of crizotinib in lung cancers harboring a MET exon 14 alteration. Nat Med. 2020;26(1):47-51.

[10] Baldacci S, Kherrouche Z, Descarpentries C, Wislez M, Dansin E, Furlan A, et al. [MET exon 14 splicing sites mutations: A new therapeutic opportunity in lung cancer]. Rev Mal Respir. 2018;35(8):796-812.

[11] Tong JH, Yeung SF, Chan AW, Chung LY, Chau SL, Lung RW, et al. MET Amplification and Exon 14 Splice Site Mutation Define Unique Molecular Subgroups of Non-Small Cell Lung Carcinoma with Poor Prognosis. Clin Cancer Res. 2016;22(12):3048-56.

[12] Cappuzzo F, Marchetti A, Skokan M, Rossi E, Gajapathy S, Felicioni L, et al. Increased MET gene copy number negatively affects survival of surgically resected non-small-cell lung cancer patients. J Clin Oncol. 2009;27(10):1667-74.

[13] Comoglio PM, Trusolino L, Boccaccio C. Known and novel roles of the MET oncogene in cancer: a coherent approach to targeted therapy. Nat Rev Cancer. 2018;18(6):341-58.

**Claims**

1. A nanobody or antigen-binding fragment thereof capable of specifically binding to c-MET, wherein the nanobody or antigen-binding fragment thereof comprises: CDR1, CDR2, and CDR3 contained in the VHH as set forth in any one of SEQ ID NOs: 1, 14 and 15;
preferably, the CDRs are defined according to the Kabat, Chothia, or IMGT numbering system.

2. The nanobody or antigen-binding fragment thereof according to claim 1, comprising:

(a) CDR1 as set forth in SEQ ID NO: 2, CDR2 as set forth in SEQ ID NO: 3, and CDR3 as set forth in SEQ ID NO: 4, wherein the CDRs are defined according to the Kabat numbering system;
or,
(b) CDR1 as set forth in SEQ ID NO: 5, CDR2 as set forth in SEQ ID NO: 6, and CDR3 as set forth in SEQ ID NO: 7, wherein the CDRs are defined according to the Chothia numbering system;
or,
(c) CDR1 as set forth in SEQ ID NO: 8, CDR2 as set forth in SEQ ID NO: 9, and CDR3 as set forth in SEQ ID NO: 10, wherein the CDRs are defined according to the IMGT numbering system.

3. The nanobody or antigen-binding fragment thereof according to claim 1 or 2, wherein the nanobody or antigen-binding fragment comprises the sequence as set forth in SEQ ID NO: 1, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto.

4. The nanobody or antigen-binding fragment thereof according to claim 1 or 2, wherein the nanobody or antigen-binding fragment is humanized;

    preferably, the nanobody or antigen-binding fragment further comprises a heavy chain framework region of a human immunoglobulin (e.g., a heavy chain framework region contained in the amino acid sequence encoded by a human heavy chain germline antibody gene), the heavy chain framework region optionally comprises one or more (e.g., 1, 2, 3, 4, 5, 6, 7, 8, 9, or 10) reversion mutations from human residues to camel residues.

5. The nanobody or antigen-binding fragment thereof according to claim 4, wherein the nanobody or antigen-binding fragment comprises the sequence as set forth in SEQ ID NO: 14 or 15, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto.

6. A polypeptide construct capable of specifically binding to c-MET, comprising the nanobody or antigen-binding fragment thereof according to any one of claims 1 to 5, and an immunoglobulin Fc domain;

    preferably, the immunoglobulin Fc domain is optionally linked to the N-terminus and/or C-terminus (e.g., C-terminus) of the nanobody or antigen-binding fragment thereof via a peptide linker;
    preferably, the immunoglobulin Fc domain is an Fc domain of IgG (e.g., an Fc domain of IgG1, IgG2, IgG3, or IgG4);
    preferably, the immunoglobulin Fc domain comprises the sequence as set forth in SEQ ID NO: 16;
    preferably, the polypeptide construct comprises the sequence as set forth in any one of SEQ ID NOs: 11-13.

7. An isolated nucleic acid molecule, encoding the nanobody or antigen-binding fragment thereof according to any one of claims 1 to 5 or the polypeptide construct according to claim 6.

8. A vector, comprising the nucleic acid molecule according to claim 7; preferably, the vector is a cloning vector or an expression vector.

9. A host cell, comprising the isolated nucleic acid molecule according to claim 7 or the vector according to claim 8.

10. A method for preparing the nanobody or antigen-binding fragment thereof according to any one of claims 1 to 5 or the polypeptide construct according to claim 6, comprising culturing the host cell according to claim 9 under conditions allowing protein expression, and recovering the nanobody or antigen-binding fragment or the polypeptide construct from the cultured host cell culture.

11. A bispecific or multispecific antibody, comprising the nanobody or antigen-binding fragment thereof according to any one of claims 1 to 5 or the polypeptide construct according to claim 6;

    preferably, the bispecific or multispecific antibody is capable of specifically binding to c-MET, and additionally specifically binding to one or more other targets;
    preferably, the bispecific or multispecific antibody further comprises at least one secondary antibody having a second binding specificity against a second target.

12. A conjugate, comprising the nanobody or antigen-binding fragment thereof according to any one of claims 1 to 5, the polypeptide construct according to claim 6, or the bispecific or multispecific antibody according to claim 11, and a conjugate moiety linked thereto;

    preferably, the conjugate moiety is selected from therapeutic agents (e.g., cytotoxic agent, cytokine, toxin, or radionuclide).

13. A pharmaceutical composition, comprising the nanobody or antigen-binding fragment thereof according to any one of claims 1 to 5, the polypeptide construct according to claim 6, the isolated nucleic acid molecule according to claim 7, the vector according to claim 8, the host cell according to claim 9, the bispecific or multispecific antibody according to claim 11, or the conjugate according to claim 12, and a pharmaceutically acceptable carrier and/or excipient;

    preferably, the pharmaceutical composition further comprises an additional pharmaceutically active agent, such as an antitumor agent.

14. Use of the nanobody or antigen-binding fragment thereof according to any one of claims 1 to 5, the polypeptide construct according to claim 6, the isolated nucleic acid molecule according to claim 7, the vector according to claim 8, the host cell according to claim 9, the bispecific or multispecific antibody according to claim 11, the conjugate according to claim 12, or the pharmaceutical composition according to claim 13, in the manufacture of a medicament for the prevention and/or treatment of a tumor in a subject;

preferably, the tumor is a c-MET positive tumor;
preferably, the tumor is a tumor with abnormal activation of c-MET pathway;
preferably, the tumor is selected from the group consisting of solid tumors, such as gastric cancer, lung cancer, liver cancer, head and neck cancer, skin cancer, colorectal cancer, ovarian cancer, esophageal cancer, pancreatic cancer, cervical cancer, mesothelioma, breast cancer, colorectal cancer, or oral squamous cell carcinoma;
preferably, the subject is a mammal, such as a human;
preferably, the nanobody or antigen-binding fragment thereof, polypeptide construct, isolated nucleic acid molecule, vector, host cell, bispecific or multispecific antibody, conjugate, or pharmaceutical composition is used alone or in combination with an additional pharmaceutically active agent (e.g., an antitumor agent).

15. A method for preventing and/or treating a tumor in a subject, comprising: administering to the subject in need thereof an effective amount of the nanobody or antigen-binding fragment thereof according to any one of claims 1 to 5, the polypeptide construct according to claim 6, the isolated nucleic acid molecule according to claim 7, the vector according to claim 8, the host cell according to claim 9, the bispecific or multispecific antibody according to claim 11, the conjugate according to claim 12, or the pharmaceutical composition according to claim 13;

preferably, the tumor is a c-MET-positive tumor;
preferably, the tumor is a tumor with abnormal activation of c-MET pathway;
preferably, the tumor is selected from the group consisting of solid tumors, such as gastric cancer, lung cancer, liver cancer, head and neck cancer, skin cancer, colorectal cancer, ovarian cancer, esophageal cancer, pancreatic cancer, cervical cancer, mesothelioma, breast cancer, colorectal cancer, or oral squamous cell carcinoma;
preferably, the subject is a mammal, such as a human.

16. A kit, comprising the nanobody or antigen-binding fragment thereof according to any one of claims 1 to 5 or the polypeptide construct according to claim 6;

preferably, the kit comprises a conjugate, and the conjugate comprises the nanobody or antigen-binding fragment thereof according to any one of claims 1 to 5 or the polypeptide construct according to claim 6, and a detectable label, such as an enzyme (e.g., horseradish peroxidase or alkaline phosphatase), a chemiluminescent reagent (e.g., acridinium ester, luminol and its derivative, or ruthenium derivative), or a fluorescent dye (e.g., fluorescein or fluorescent protein), a radionuclide, or biotin, which is linked to the nanobody or antigen-binding fragment thereof or the polypeptide construct;
preferably, the kit comprises the nanobody or antigen-binding fragment thereof according to any one of claims 1 to 5 or the polypeptide construct according to claim 6, and a secondary antibody capable of specifically recognizing the nanobody or antigen-binding fragment thereof or the polypeptide construct; optionally, the secondary antibody further comprises a detectable label, such as an enzyme (e.g., horseradish peroxidase or alkaline phosphatase), a chemiluminescent reagent (e.g., acridinium ester, luminol and its derivative, or ruthenium derivative), a fluorescent dye (e.g., fluorescein or fluorescent protein), a radionuclide, or biotin.

17. A method for detecting the presence or level of c-MET in a sample, comprising using the nanobody or antigen-binding fragment thereof according to any one of claims 1 to 5 or the polypeptide construct according to claim 6;
preferably, the method is an immunological assay, such as Western blotting, enzyme immunoassay (e.g., ELISA), chemiluminescent immunoassay, fluorescence immunoassay, or radioimmunoassay.

18. A method for detecting whether a tumor can be treated with an anti-tumor therapy targeting c-MET, comprising detecting the presence or level of c-MET in a sample from a subject using the nanobody or antigen-binding fragment thereof according to any one of claims 1 to 5 or the polypeptide construct according to claim 6;

preferably, the method is an immunological assay, such as Western blotting, enzyme immunoassay (e.g., ELISA), chemiluminescent immunoassay, fluorescence immunoassay, or radioimmunoassay;

preferably, the sample is a cell sample (e.g., a sample containing tumor cells) or a body fluid sample (e.g., blood) from the subject (e.g., a mammal, such as a human).

19. Use of the nanobody or antigen-binding fragment thereof according to any one of claims 1 to 5 or the polypeptide construct according to claim 6 in the manufacture of a diagnostic reagent for detecting the presence or level of c-MET in a sample or for detecting whether a tumor can be treated with an anti-tumor therapy targeting c-MET;

preferably, the sample is a cell sample (e.g., a sample containing tumor cells) or a body fluid sample (e.g., blood) from a subject (e.g., a mammal, such as a human).

Fig. 1

Fig. 2

Fig. 3A

Fig. 3B

Fig. 4

Fig. 5

Fig. 6

Fig. 7A

Fig. 7B

Fig 8

Fig. 9

## INTERNATIONAL SEARCH REPORT

| International application No. |
| --- |
| **PCT/CN2024/111721** |

| A. | CLASSIFICATION OF SUBJECT MATTER |
| --- | --- |

C07K16/40(2006.01)i; C12N15/13(2006.01)i; C12N15/70(2006.01)i; A61K49/00(2006.01)i; G01N33/573(2006.01)i

According to International Patent Classification (IPC) or to both national classification and IPC

| B. | FIELDS SEARCHED |
| --- | --- |

Minimum documentation searched (classification system followed by classification symbols)

    IPC: C07K, C12N, A61K, G01N

Documentation searched other than minimum documentation to the extent that such documents are included in the fields searched

Electronic data base consulted during the international search (name of data base and, where practicable, search terms used)

    DWPI, VEN, CNABS, CNTXT, CNKI, PubMed, GenBank, EBI, STN, WPABSC, WPABS, ENTXT, ENTXTC, Wanfang Database, ISI web of Knowledge: C-MET, 纳米抗体, VHH, nano antibod+, nanobod+, Nb, 酪氨酸激酶受体, 细胞间质上皮转换因子, 肝细胞生长因子受体, MET-H5227, cellular-mesenchymal epithelial transition factor, hepatocyte growth factor receptor, HGFR, 双特异抗体, 嵌合抗体, 明慧医药

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 110770254 A (CHONG KUN DANG PHARMACEUTICAL CORP.) 07 February 2020 (2020-02-07)<br>description, embodiments | 1-19 |
| A | CN 114702590 A (XIDIAN UNIVERSITY) 05 July 2022 (2022-07-05)<br>claims 1-8 | 1-19 |
| A | WO 2023078393 A1 (CHIA TAI TIANQING PHARMACEUTICAL GROUP CO., LTD.) 11 May 2023 (2023-05-11)<br>claims 1-18 | 1-19 |
| A | WO 2023078391 A1 (CHIA TAI TIANQING PHARMACEUTICAL GROUP CO., LTD.) 11 May 2023 (2023-05-11)<br>claims 1-18 | 1-19 |
| A | CN 116496400 A (THE SECOND HOSPITAL, LANZHOU UNIVERSITY) 28 July 2023 (2023-07-28)<br>description, embodiments | 1-19 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **13 September 2024** | **24 October 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/111721** |

### C. DOCUMENTS CONSIDERED TO BE RELEVANT

| Category* | Citation of document, with indication, where appropriate, of the relevant passages | Relevant to claim No. |
| --- | --- | --- |
| A | WO 2023122588 A2 (FUSION PHARMACEUTICALS INC. et al.) 29 June 2023 (2023-06-29) description, embodiments | 1-19 |
| A | WO 2011020925 A1 (PIERRE FABRE MEDICAMENT) 24 February 2011 (2011-02-24) claims 1-25 | 1-19 |
| A | WO 2007126799 A2 (NOVARTIS AG) 08 November 2007 (2007-11-08) description, embodiments | 1-19 |

Form PCT/ISA/210 (second sheet) (July 2022)

**INTERNATIONAL SEARCH REPORT**

| International application No. |
| --- |
| **PCT/CN2024/111721** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

International application No.

**PCT/CN2024/111721**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **15, 18**
   because they relate to subject matter not required to be searched by this Authority, namely:

   Claim 15 relates to a method for preventing and/or treating tumors in a subject, and claim 18 relates to a method for detecting whether a tumor can be treated by means of an anti-tumor therapy targeting c-METD, that is, claims 15 and 18 relate to subject matter for which no search is required by the International Searching Authority as defined in PCT Rule 39.1: (4) methods for treatment of the human or animal body by surgery or therapy, as well as diagnostic methods. The international search is performed on the basis of the use of the nanobody in the preparation of a drug for preventing and treating tumors.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
|---|
| **PCT/CN2024/111721** |

| Patent document cited in search report | | | Publication date (day/month/year) | Patent family member(s) | | | Publication date (day/month/year) |
|---|---|---|---|---|---|---|---|
| CN | 110770254 | A | 07 February 2020 | MY | 192630 | A | 29 August 2022 |
| | | | | US | 2020231681 | A1 | 23 July 2020 |
| | | | | US | 11479612 | B2 | 25 October 2022 |
| | | | | AU | 2018278730 | A1 | 14 November 2019 |
| | | | | AU | 2018278730 | B2 | 04 February 2021 |
| | | | | MX | 2019014316 | A | 27 January 2020 |
| | | | | PH | 12019550233 | A1 | 20 July 2020 |
| | | | | CA | 3061704 | A1 | 06 December 2018 |
| | | | | CA | 3061704 | C | 23 January 2024 |
| | | | | NZ | 758605 | A | 29 July 2022 |
| | | | | KR | 20180131470 | A | 10 December 2018 |
| | | | | KR | 102078292 | B1 | 19 February 2020 |
| | | | | EP | 3630844 | A1 | 08 April 2020 |
| | | | | EP | 3630844 | A4 | 03 March 2021 |
| | | | | RU | 2019143101 | A3 | 30 June 2021 |
| | | | | RU | 2019143101 | A | 16 July 2021 |
| | | | | JP | 2020522258 | A | 30 July 2020 |
| | | | | JP | 7325339 | B2 | 14 August 2023 |
| | | | | BR | 112019025070 | A2 | 23 March 2021 |
| | | | | TW | 201900678 | A | 01 January 2019 |
| | | | | TWI | 707869 | B | 21 October 2020 |
| CN | 114702590 | A | 05 July 2022 | None | | | |
| WO | 2023078393 | A1 | 11 May 2023 | None | | | |
| WO | 2023078391 | A1 | 11 May 2023 | KR | 20240099302 | A | 28 June 2024 |
| | | | | TW | 202325729 | A | 01 July 2023 |
| CN | 116496400 | A | 28 July 2023 | None | | | |
| WO | 2023122588 | A2 | 29 June 2023 | AU | 2022419564 | A1 | 04 July 2024 |
| | | | | AR | 128052 | A1 | 20 March 2024 |
| | | | | CO | 2024008733 | A2 | 29 July 2024 |
| | | | | WO | 2023122588 | A3 | 21 September 2023 |
| | | | | TW | 202333799 | A | 01 September 2023 |
| | | | | IL | 313333 | A | 01 August 2024 |
| WO | 2011020925 | A1 | 24 February 2011 | BR | 112012003759 | A2 | 11 July 2017 |
| | | | | ZA | 201202076 | B | 28 November 2012 |
| | | | | AU | 2010284944 | A1 | 08 March 2012 |
| | | | | AU | 2010284944 | B2 | 28 January 2016 |
| | | | | RU | 2012109004 | A | 27 September 2013 |
| | | | | RU | 2582265 | C2 | 20 April 2016 |
| | | | | IN | DEN2012 | A | 05 June 2015 |
| | | | | EP | 2467402 | A1 | 27 June 2012 |
| | | | | EP | 2467402 | B1 | 01 August 2018 |
| | | | | NZ | 598194 | A | 29 May 2015 |
| | | | | US | 2014295452 | A1 | 02 October 2014 |
| | | | | JP | 2013502213 | A | 24 January 2013 |
| | | | | JP | 5951486 | B2 | 13 July 2016 |
| | | | | KR | 20120051734 | A | 22 May 2012 |
| | | | | NZ | 700437 | A | 29 April 2016 |
| | | | | ES | 2692522 | T3 | 04 December 2018 |
| | | | | SG | 178339 | A1 | 29 March 2012 |

Form PCT/ISA/210 (patent family annex) (July 2022)

# EP 4 763 865 A1

## INTERNATIONAL SEARCH REPORT
### Information on patent family members

| International application No. |
| --- |
| **PCT/CN2024/111721** |

| Patent document cited in search report | | | Publication date (day/month/year) | Patent family member(s) | | | Publication date (day/month/year) |
| --- | --- | --- | --- | --- | --- | --- | --- |
| | | | | EP | 2287197 | A1 | 23 February 2011 |
| | | | | CA | 2769427 | A1 | 24 February 2011 |
| | | | | CA | 2769427 | C | 10 March 2020 |
| | | | | TW | 201111781 | A | 01 April 2011 |
| | | | | AR | 077901 | A1 | 28 September 2011 |
| | | | | MX | 2012002139 | A | 07 March 2012 |
| | | | | IL | 218202 | A0 | 30 April 2012 |
| | | | | IL | 218202 | A | 31 July 2017 |
| | | | | US | 2012149031 | A1 | 14 June 2012 |
| | | | | US | 8673302 | B2 | 18 March 2014 |
| WO | 2007126799 | A2 | 08 November 2007 | AU | 2007245181 | A1 | 08 November 2007 |
| | | | | PE | 20080352 | A1 | 12 June 2008 |
| | | | | BRPI | 0709917 | A2 | 05 July 2011 |
| | | | | MX | 2008012485 | A | 10 October 2008 |
| | | | | KR | 20080113218 | A | 29 December 2008 |
| | | | | CL | 2007000851 | A1 | 14 March 2008 |
| | | | | US | 2009175860 | A1 | 09 July 2009 |
| | | | | US | 8101727 | B2 | 24 January 2012 |
| | | | | CA | 2646048 | A1 | 08 November 2007 |
| | | | | TW | 200815470 | A | 01 April 2008 |
| | | | | RU | 2008142833 | A | 10 May 2010 |
| | | | | AR | 060241 | A1 | 04 June 2008 |
| | | | | WO | 2007126799 | A3 | 03 April 2008 |
| | | | | EP | 2004693 | A2 | 24 December 2008 |
| | | | | EP | 2004693 | B1 | 06 June 2012 |
| | | | | JP | 2009532026 | A | 10 September 2009 |
| | | | | JP | 5536445 | B2 | 02 July 2014 |
| | | | | ES | 2386738 | T3 | 28 August 2012 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311018785

### Non-patent literature cited in the description

- Fundamental Immunology. Raven Press, 1989
- **LEFRANC et al.** *Dev. Comparat. Immunol.*, 2003, vol. 27, 55-77
- **KABAT et al.** Sequences of Proteins of Immunological Interest. Public Health Service, National Institutes of Health, 1991
- **CHOTHIA** ; **LESK**. *J. Mol. Biol.*, 1987, vol. 196, 901-917
- **CHOTHIA et al.** *Nature*, 1989, vol. 342, 878-833
- **MALMQVIST M**. *Nature*, 1993, vol. 361, 186-187
- **DAVIES et al.** *Annual Rev Biochem*, 1990, vol. 59, 439-473
- **NEEDLEMAN et al.** *J. Mol. Biol.*, 1970, vol. 48, 443-453
- **E. MEYERS** ; **W. MILLER**. *Comput. Appl. Biosci.*, 1988, vol. 4, 11-17
- **NEEDLEMAN** ; **WUNSCH**. *J Mol Biol.*, 1970, vol. 48, 444-453
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc. Natl Acad. Set USA*, 1997, vol. 94, 412-417
- Immunology-A Synthesis. Sinauer Associates, 1991
- Remington's Pharmaceutical Sciences. Mack Publishing Company, 1995
- **GHERARDI E** ; **BIRCHMEIER W** ; **BIRCHMEIER C** ; **VANDE WOUDE G**. Targeting MET in cancer: rationale and progress.. *Nat Rev Cancer.*, 2012, vol. 12 (2), 89-103
- **STOKER M** ; **GHERARDI E** ; **PERRYMAN M** ; **GRAY J**. Scatter factor is a fibroblast-derived modulator of epithelial cell mobility.. *Nature*, 1987, vol. 327 (6119), 239-42
- **KONG-BELTRAN M** ; **STAMOS J** ; **WICKRAMASINGHE D**. The Sema domain of Met is necessary for receptor dimerization and activation.. *Cancer Cell.*, 2004, vol. 6 (1), 75-84

- **GHERARDI E** ; **SANDIN S** ; **PETOUKHOV MV** ; **FINCH J** ; **YOULES ME** ; **OFVERSTEDT LG et al.** Structural basis of hepatocyte growth factor/scatter factor and MET signaling.. *Proc Natl Acad Sci U S A.*, 2006, vol. 103 (11), 4046-51
- **UCHIKAWA E** ; **CHEN Z** ; **XIAO GY** ; **ZHANG X** ; **BAI XC**. Structural basis of the activation of c-MET receptor.. *Nat Commun.*, 2021, vol. 12 (1), 4074
- **PONZETTO C** ; **BARDELLI A** ; **ZHEN Z** ; **MAINA F** ; **DALLA ZONCA P** ; **GIORDANO S et al.** A multifunctional docking site mediates signaling and transformation by the hepatocyte growth factor/scatter factor receptor family.. *Cell.*, 1994, vol. 77 (2), 261-71
- **FASOLO A** ; **SESSA C** ; **GIANNI L** ; **BROGGINI M**. Seminars in clinical pharmacology: an introduction to MET inhibitors for the medical oncologist.. *Ann Oncol.*, 2013, vol. 24 (1), 14-20
- **TRUSOLINO L** ; **BERTOTTI A** ; **COMOGLIO PM**. MET signaling: principles and functions in development, organ regeneration and cancer.. *Nat Rev Mol Cell Biol.*, 2010, vol. 11 (12), 834-48
- **DRILON A** ; **CLARK JW** ; **WEISS J** ; **OU SI** ; **CAMIDGE DR** ; **SOLOMON BJ et al.** Antitumor activity of crizotinib in lung cancers harboring a MET exon 14 alteration.. *Nat Med.*, 2020, vol. 26 (1), 47-51
- **BALDACCI S** ; **KHERROUCHE Z** ; **DESCARPENTRIES C** ; **WISLEZ M** ; **DANSIN E** ; **FURLAN A et al.** MET exon 14 splicing sites mutations: A new therapeutic opportunity in lung cancer].. *Rev Mal Respir.*, 2018, vol. 35 (8), 796-812
- **TONG JH** ; **YEUNG SF** ; **CHAN AW** ; **CHUNG LY** ; **CHAU SL** ; **LUNG RW et al.** MET Amplification and Exon 14 Splice Site Mutation Define Unique Molecular Subgroups of Non-Small Cell Lung Carcinoma with Poor Prognosis.. *Clin Cancer Res.*, 2016, vol. 22 (12), 3048-56

- **CAPPUZZO F ; MARCHETTI A ; SKOKAN M ; ROSSI E ; GAJAPATHY S ; FELICIONI L et al.** Increased MET gene copy number negatively affects survival of surgically resected non-small-cell lung cancer patients.. *J Clin Oncol.*, 2009, vol. 27 (10), 1667-74

- **COMOGLIO PM ; TRUSOLINO L ; BOCCACCIO C**. Known and novel roles of the MET oncogene in cancer: a coherent approach to targeted therapy.. *Nat Rev Cancer.*, 2018, vol. 18 (6), 341-58